(19) Europäisches Patentamt
European Patent Office
Office européen des brevets

(11) EP 4 357 342 A1

# (12) EUROPEAN PATENT APPLICATION
published in accordance with Art. 153(4) EPC

(43) Date of publication:
**24.04.2024 Bulletin 2024/17**

(21) Application number: **22866556.8**

(22) Date of filing: **05.09.2022**

(51) International Patent Classification (IPC):
***C07D 413/12*** *(2006.01)* ***C07D 207/27*** *(2006.01)*
***A61K 31/4015*** *(2006.01)* ***A61P 31/00*** *(2006.01)*

(52) Cooperative Patent Classification (CPC):
**A61K 31/4015; A61K 31/427; A61K 31/472; A61K 31/4725; A61K 31/496; A61K 31/513; A61K 31/635; A61K 38/06; A61P 31/00; A61P 31/14; C07D 207/27; C07D 413/12; C07K 5/0804; C07K 5/0812**

(86) International application number:
**PCT/CN2022/117124**

(87) International publication number:
**WO 2023/036093 (16.03.2023 Gazette 2023/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.09.2021 CN 202111057236**
**03.11.2021 CN 202111296533**
**15.02.2022 CN 202210138841**
**05.07.2022 CN 202210793838**
**16.08.2022 CN 202210982186**

(71) Applicant: **Guangdong Raynovent Biotech Co., Ltd.**
**Huangpu District**
**Guangzhou 510700 (CN)**

(72) Inventors:
- **CHEN, Xiaoxin**
  **Guangzhou, Guangdong 510700 (CN)**
- **WANG, Jingjing**
  **Guangzhou, Guangdong 510700 (CN)**
- **HUANG, Jianzhou**
  **Guangzhou, Guangdong 510700 (CN)**
- **LIU, Zhuowei**
  **Guangzhou, Guangdong 510700 (CN)**
- **LONG, Chaofeng**
  **Guangzhou, Guangdong 510700 (CN)**
- **CHEN, Shuhui**
  **Guangzhou, Guangdong 510700 (CN)**
- **CHEN, Kevin X**
  **Guangzhou, Guangdong 510700 (CN)**

(74) Representative: **Potter Clarkson**
**Chapel Quarter**
**Mount Street**
**Nottingham NG1 6HQ (GB)**

## (54) KETOAMIDE DERIVATIVE AND APPLICATION THEREOF

(57) A class of ketoamide derivatives and an application thereof are disclosed. Specifically disclosed are a compound of formula (IV) and a pharmaceutically acceptable salt thereof.

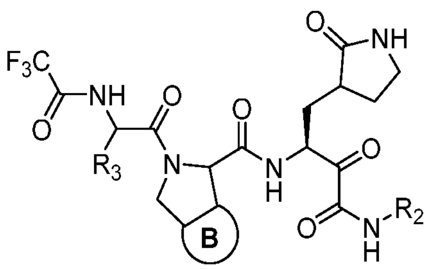

**( IV)**

EP 4 357 342 A1

## Description

### TECHNICAL FIELD

The present application relates to a class of ketoamide derivatives and an application thereof, and particularly to a compound of formula (IV) and a pharmaceutically acceptable salt thereof.

### BACKGROUND

Atypical Pneumonia Type 2 caused by a novel coronavirus (SARS-Cov-2), which began in December 2019, rapidly swept the globe, posing an unprecedented challenge to human health and social development.

The major protease of coronaviruses, also known as 3CL protease, is a key protein in viral replication, and its main function is to hydrolyze two polyproteins expressed by the virus. It is suggested by sequence analysis that the 3CL protease has the potential to be one of the key targets for drug design. Blocking the replication process of coronaviruses by developing their inhibitors is of great value and significance for the prevention and treatment of coronavirus infections.

PF-07321332 is a potent orally active inhibitor of SARS-CoV 3CL PRO, having a structure shown as below:

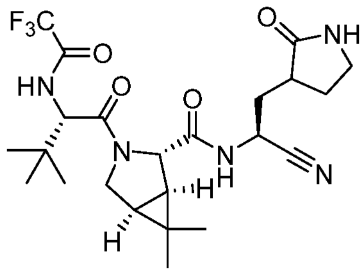

**PF-07321332**

### SUMMARY OF THE INVENTION

The present application provides a compound of formula (IV) or a pharmaceutically acceptable salt thereof,

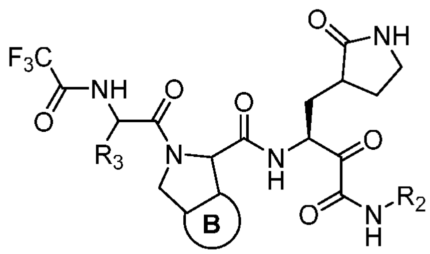

**( IV)**

wherein,

$R_3$ is selected from

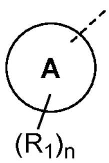

and $R_4$;

$R_1$ is each independently selected from F, Cl, Br, I, $OR_{11}$, CN, $CH_3S(O)_m$- and $NHR_{12}$ and $C_{1-3}$ alkyl, and the $C_{1-3}$

alkyl is optionally substituted with 1, 2 or 3 F;

$R_{11}$ is selected from H, $C_{1-3}$ alkyl, $CH_3(OCH_2CH2)_p$- and $H(OCH_2CH_2)_q$-, and the $C_{1-3}$ alkyl is optionally substituted with 1, 2 or 3 F;

$R_{12}$ is selected from $C_{1-3}$ alkyl, $CH_3CO$- and $CH_3SO_2$-, and the $C_{1-3}$ alkyl, $CH_3CO$- and $CH_3SO_2$- are optionally and independently substituted with 1, 2 or 3 F;

m is selected from 0, 1 and 2;

p and q are selected from 1, 2, 3, 4, 5, and 6;

n is selected from 0, 1, 2, 3, and 4;

$R_2$ is selected from $C_{1-4}$ alkyl, $C_{3-6}$ cycloalkyl, and benzyl, and the $C_{1-4}$ alkyl, $C_{3-6}$ cycloalkyl, and benzyl are optionally substituted with 1, 2 or 3 F;

$R_4$ is selected from $C_{1-8}$ alkyl optionally substituted with 1, 2 or 3 F;

ring A is selected from $C_{3-10}$ cycloalkyl, 3-10-membered heterocycloalkyl, and phenyl;

ring B is selected from $C_{3-8}$ cycloalkyl and 5-membered heterocycloalkyl, and the $C_{3-8}$ cycloalkyl and 5-membered heterocycloalkyl are optionally substituted with 1 or 2 $R_a$;

$R_a$ is each independently selected from H and $C_{1-3}$ alkyl; and

the "5-membered heterocycloalkyl" includes 1, 2 or 3 heteroatoms or atomic groups independently selected from O, S, $SO_2$, N, P, and Se.

In some embodiments of the present application, the above $R_1$ is selected from F and methyl, with other variables being as defined in the present application.

In some embodiments of the present application, the above ring A is selected from

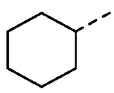,

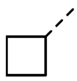, 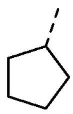, 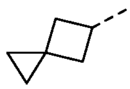, and 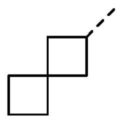,

with other variables being as defined in the present application.

In some embodiments of the present application, the above structural unit

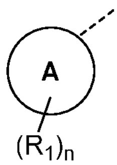

is selected from

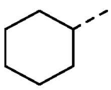, 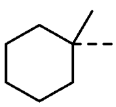, 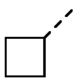, 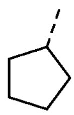, 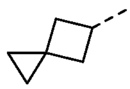, and 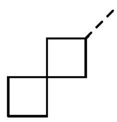,

with other variables being as defined in the present application.

In some embodiments of the present application, the above $R_4$ is selected from t-butyl, with other variables being as defined in the present application.

In some embodiments of the present application, the above $R_a$ is selected from H and methyl, with other variables being as defined in the present application.

In some embodiments of the present application, the above ring B is selected from

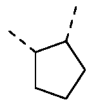

and

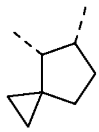,

with other variables being as defined in the present application.

In some embodiments of the present application, the above structural unit

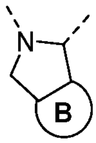

is selected from

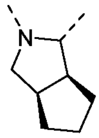, 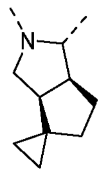, and 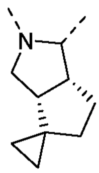,

with other variables being as defined in the present application.

The present application provides a compound selected from the following formula or a pharmaceutically acceptable salt thereof,

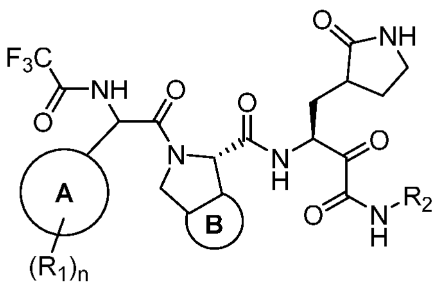

**(I)**

wherein,

- $R_1$ is each independently selected from F, Cl, Br, I, $OR_{11}$, CN, $CH_3S(O)_m$- and $NHR_{12}$ and $C_{1-3}$ alkyl, and the $C_{1-3}$ alkyl is optionally substituted with 1, 2 or 3 F;
- $R_{11}$ is selected from H, $C_{1-3}$ alkyl, $CH_3(OCH_2CH2)_p$- and $H(OCH_2CH_2)_q$-, and the $C_{1-3}$ alkyl is optionally substituted with 1, 2 or 3 F;
- $R_{12}$ is selected from $C_{1-3}$ alkyl, $CH_3CO$- and $CH_3SO_2$-, and the $C_{1-3}$ alkyl is optionally substituted with 1, 2 or 3 F;
- m is selected from 0, 1 and 2;
- p and q are selected from 1, 2, 3, 4, 5, and 6;
- n is selected from 0, 1, 2, 3, and 4;
- $R_2$ is selected from $C_{1-4}$ alkyl, $C_{3-6}$ cycloalkyl, and benzyl, and the $C_{1-4}$ alkyl, $C_{3-6}$ cycloalkyl, and benzyl are optionally

substituted with 1, 2 or 3 F;
ring A is selected from $C_{3-10}$ cycloalkyl, 3-10-membered heterocycloalkyl, and phenyl;
ring B is selected from $C_{3-6}$ cycloalkyl and 5-membered heterocycloalkyl; and the $C_{3-6}$ cycloalkyl and 5-membered heterocycloalkyl are optionally substituted with 1 or 2 $R_a$;
$R_a$ is each independently selected from H and $C_{1-3}$ alkyl;

the "5-membered heterocycloalkyl" includes 1, 2 or 3 heteroatoms or atomic groups independently selected from O, S, $SO_2$, N, P, and Se.

In some embodiments of the present application, the above $R_1$ is selected from F and methyl, with other variables being as defined in the present application.

In some embodiments of the present application, the above ring A is selected from

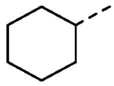,

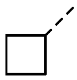, 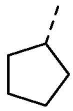, 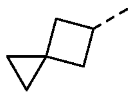, and 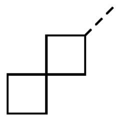

, with other variables being as defined in the present application.

In some embodiments of the present application, the above structural unit

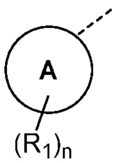

is selected from

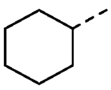, 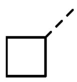, 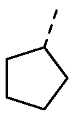, 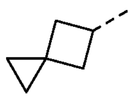, and 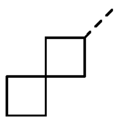,

with other variables being as defined in the present application.

In some embodiments of the present application, the above $R_a$ is selected from H and methyl, with other variables being as defined in the present application.

In some embodiments of the present application, the above structural unit

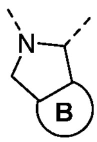

is selected from

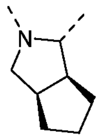

or

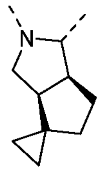

,

with other variables being as defined in the present application.

In some embodiments of the present application, the above compound is selected from structures of formulas (I-1), (IV-1) and (IV-2),

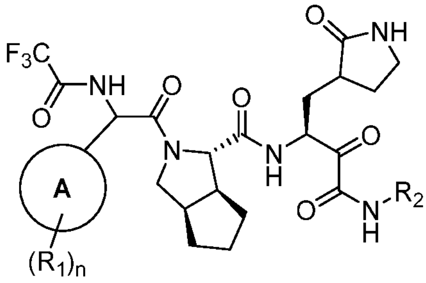

**( I-1 )**

,

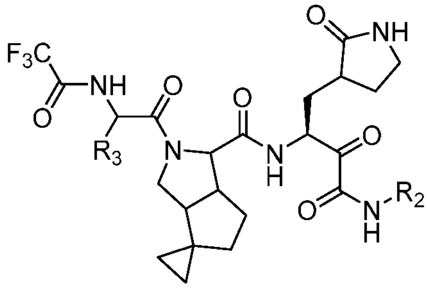

**( IV-1 )**

,

and

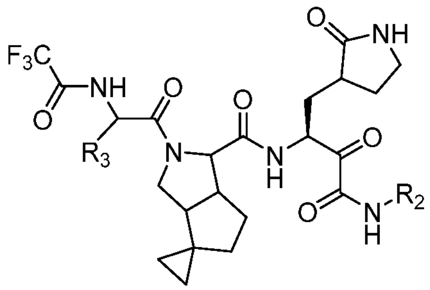

**( IV-2 )**

,

wherein, $R_1$, $R_2$, $R_3$, n and ring A are as defined in the present application.

In some embodiments of the present application, the above compound is selected from structures of formulas (I-1a), (IV-1a) and (IV-2a),

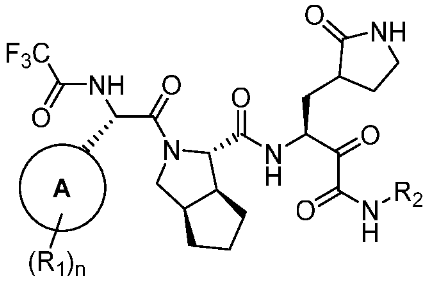

**( I-1a )**

,

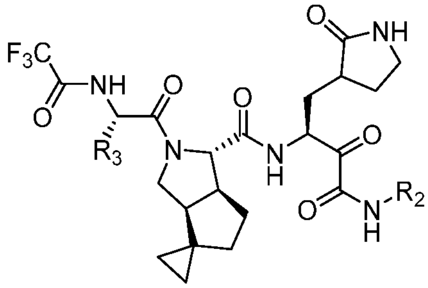

**( IV-1a )**

,

and

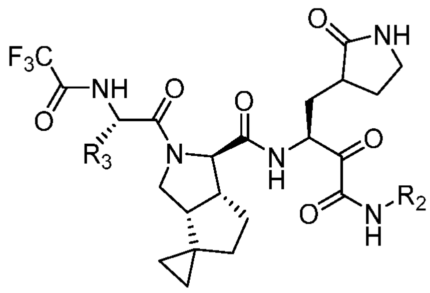

( IV-2a ) ,

wherein, $R_1$, $R_2$, $R_3$, n and ring A are as defined in the present application.

Further embodiments of the present application result from any combination of the above variables.

The present application further provides a compound selected from:

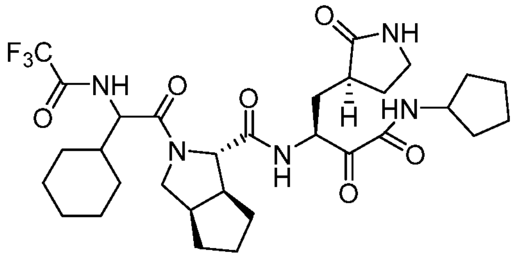
,
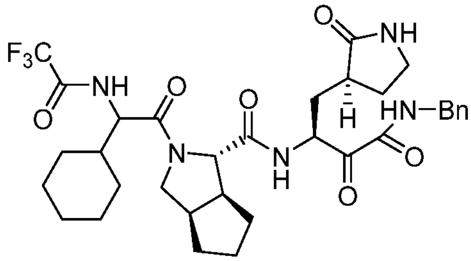
,

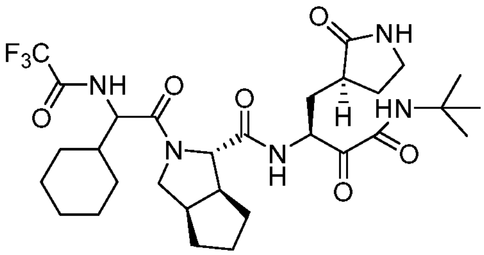
,
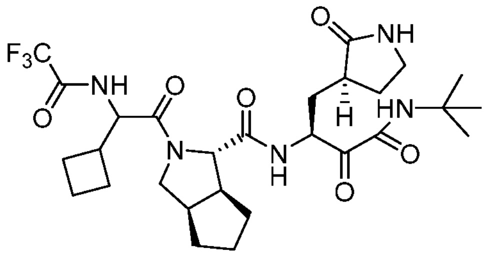
,

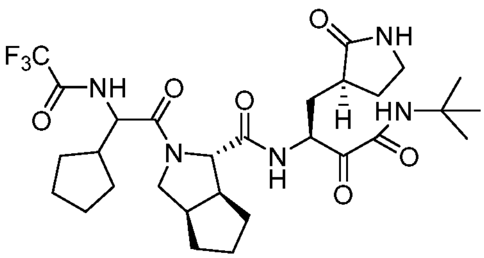
,
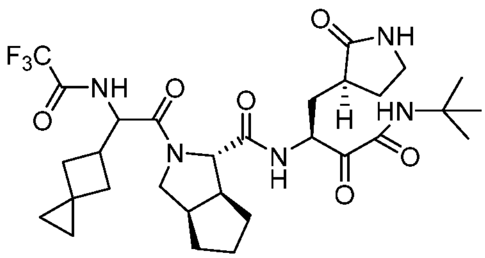
,

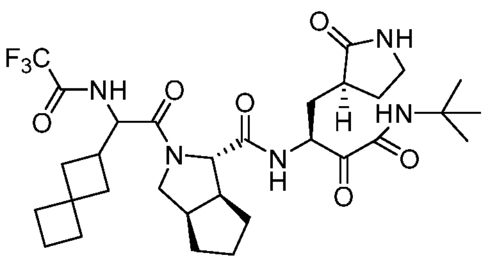
,
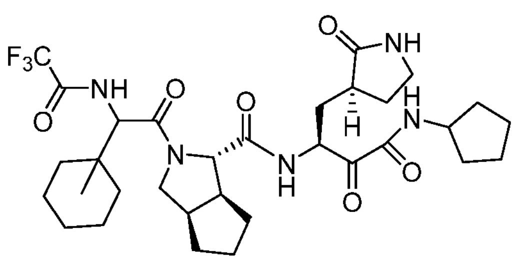
,

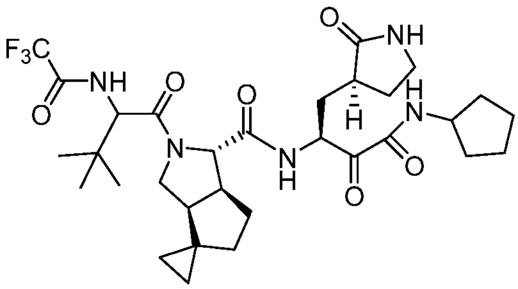

, and

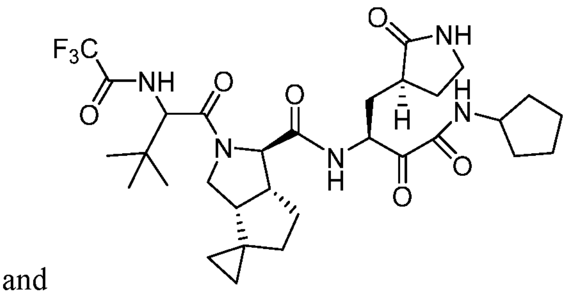

;

or a pharmaceutically acceptable salt thereof.

The present application further provides a compound selected from:

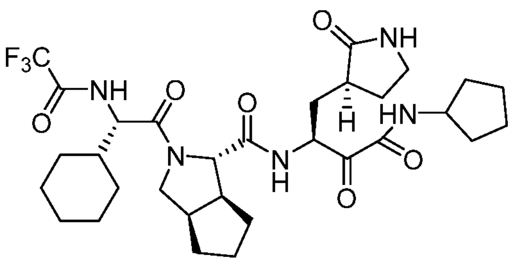

,

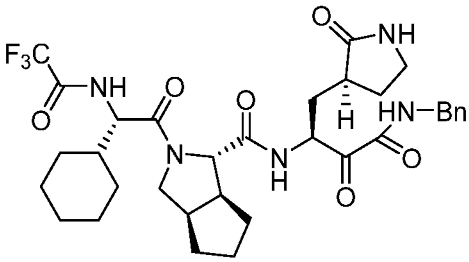

,

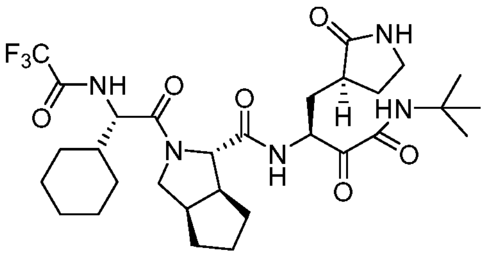

,

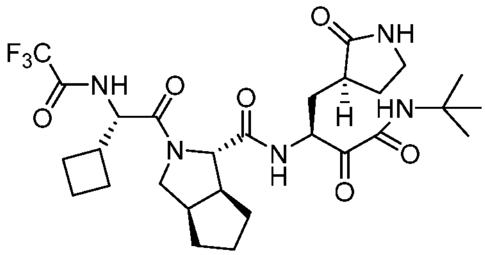

,

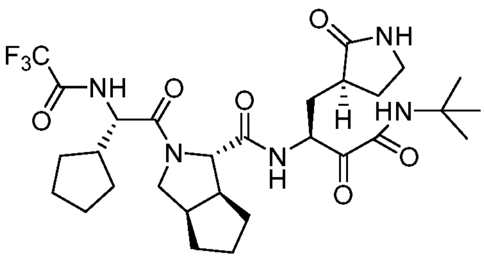

,

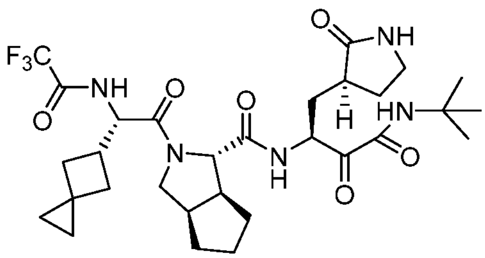

,

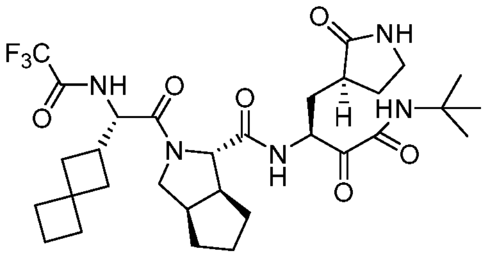

,

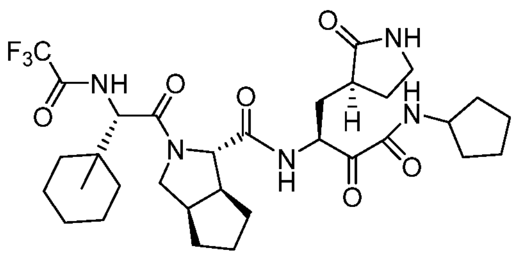

,

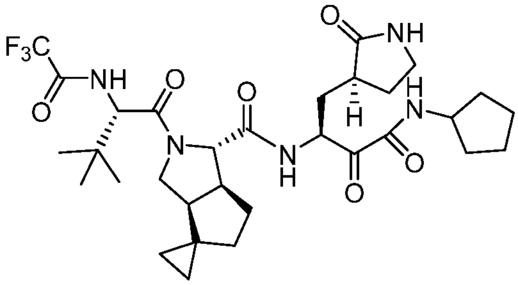
, and
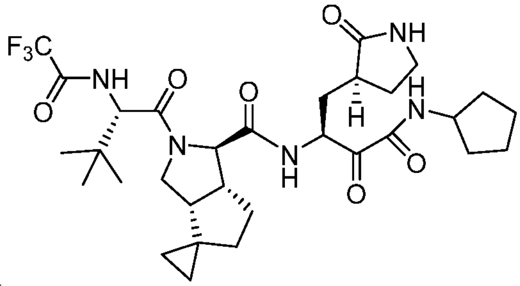
;

or a pharmaceutically acceptable salt thereof.

The present application provides a combined administration method, which includes administering to a subject in need thereof a therapeutically effective amount of the compound or the pharmaceutically acceptable salt thereof in any one embodiment of the present application, and a therapeutically acceptable dosage of other antiviral drug.

The present application further provides a method for treating a coronavirus infection, which includes administering to a subject in need thereof a therapeutically effective amount of the compound or the pharmaceutically acceptable salt thereof in any one embodiment of the present application, and a therapeutically acceptable dosage of other antiviral drug.

In some embodiments of the present application, the above other antiviral drug is selected from Ritonavir, Indinavir, Nelfinavir, Saquinavir, Amprenavir or Lopinavir. In the above method for treating a coronavirus infection, a mass ratio of the compound or the pharmaceutically acceptable salt thereof in any one embodiment of the present application to Ritonavir, Indinavir, Nelfinavir, Saquinavir, Amprenavir or Lopinavir is 1:1 to 5:1, specifically 1:1, 2:1, 3:1, 4:1 or 5:1. It is surprisingly found through experiments that the ratio within such range is conducive to achieving the synergistic effect of the two therapeutic components, thus realizing excellent comprehensive therapeutic effects. Furthermore, in the present method, the therapeutic components may be contained in the same unit preparation for administration, that is, compound preparation administration, or preparations containing different therapeutic components may be administered separately, that is, clinical combination medication.

In some embodiments of the present application, the above coronavirus infection is selected from an infection of HCoV-229E, HCoV-OC43, HCoV-NL63, HCoV-HKU1, SARS-CoV, MERS-CoV or SARS-CoV-2, or a variant thereof.

In some embodiments of the present application, the above coronavirus infection is an infection of SARS-CoV-2 or a variant thereof.

The present application further provides a synthetic route below:

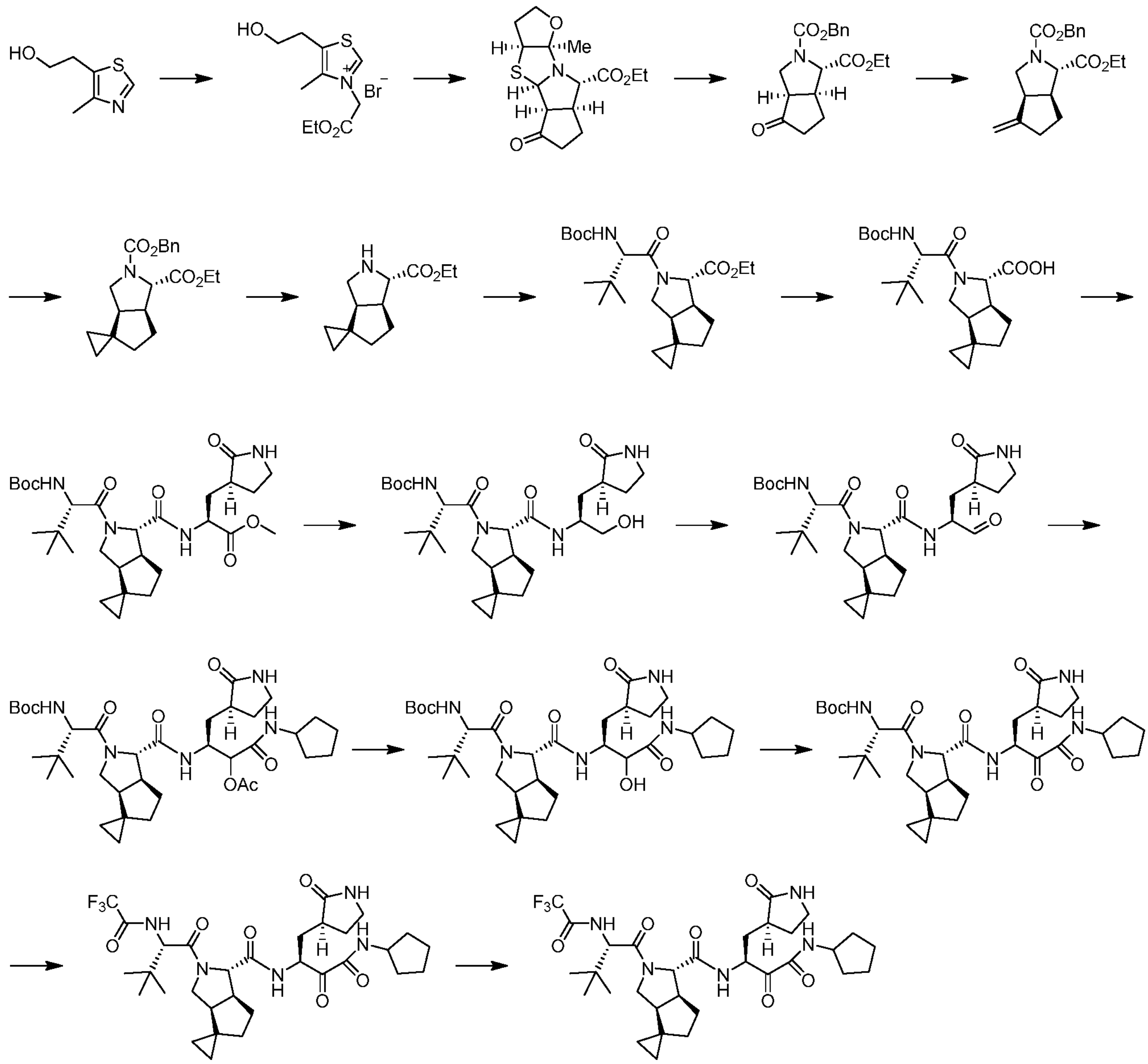

### Technical Effects

The compounds of the present application exhibit excellent in vitro inhibitory activity against Mpro protease of novel coronavirus, exhibit excellent in vitro anti-coronavirus activity at the cellular level, and are not cytotoxic. In pharmacokinetic studies, the compounds of the present application show significantly higher exposure in plasma, slower clearance rates, longer half-lives and better pharmacokinetic properties than the reference molecule PF-07321332.

### Definition and Illustration

Unless otherwise indicated, the following terms and phrases used herein are intended to have the following meanings. A particular term or phrase should not be considered indefinite or unclear in the absence of a particular definition, but should be understood in its common meaning. When a trade name is mentioned herein, it is intended to refer to its corresponding commodity or its active ingredient.

The term "pharmaceutically acceptable" as used herein refers to those compounds, materials, compositions and/or dosage forms suitable for use in contact with human and animal tissues under the reliable medical judgment, without undue toxicity, irritation, allergic response or other problems or complications, and commensurate with a reasonable benefit/risk ratio.

The term "pharmaceutically acceptable salt" refers to a salt of the compound of the present application, which is prepared from a compound having a specific substituent found in the present application and a relatively non-toxic acid or base. When the compound of the present application contains a relatively acidic functional group, a base addition

salt may be obtained by contacting a neutral form of such compound with a sufficient amount of base in a pure solution or a suitable inert solvent. Pharmaceutically acceptable base addition salts include sodium, potassium, calcium, ammonium, organic amine, or magnesium salts or similar salts. When the compound of the present application contains a relatively basic functional group, an acid addition salt may be obtained by contacting a neutral form of such compound with a sufficient amount of acid in a pure solution or a suitable inert solvent.

The pharmaceutically acceptable salt of the present application can be synthesized from parent compounds containing acid or base group through conventional chemical methods. Generally, such salt is prepared by reacting such compounds in form of free acid or base with a stoichiometric amount of an appropriate base or acid in water or an organic solvent or a mixture of both.

The term "pharmaceutical composition" refers to a composition including one or more of the compounds of the present application, isomers or pharmaceutically acceptable salts thereof, and other components, such as a physiologically/pharmaceutically usable carrier and excipient. The pharmaceutical composition is intended to facilitate the administration to a living organism and to facilitate the absorption of the active ingredient so as to exert the biological activity.

The term "therapeutically effective amount" is intended to include the amount of a compound that, when administered, is sufficient to prevent or alleviate the progression of one or more symptoms or conditions of a disease to a certain extent. The term "therapeutically effective amount" also refers to the amount of a compound that is sufficient to enable a biological or medical response of a biological molecule (e.g., a protein, enzyme, RNA, or DNA), cell, tissue, system, animal, or human to be detected. Such responses are desired by a researcher, veterinarian, medical doctor, or clinician.

Unless otherwise indicated, the term "isomer" is intended to include geometrical isomers, cis-trans isomers, stereoisomers, enantiomers, optical isomers, diastereomers, and tautomers.

The compound of the present application may exist in a specific geometric or stereoisomeric form. The present application contemplates all such compounds, including cis and trans isomers, (-)- and (+)- enantiomers, (*R*)- and (*S*)-enantiomers, diastereomers, (*D*)-isomers, (*L*)-isomers, and their racemic mixtures and other mixtures, such as enantiomerically or diastereomerically enriched mixtures, all of which are subject to the scope of the present application. Additional asymmetric carbon atoms may be present in substituents such as alkyl groups. All these isomers and mixtures thereof are all included within the scope of the present application.

Unless otherwise indicated, the term "enantiomers" or "optical isomers" refers to stereoisomers that are mirror images of each other.

Unless otherwise indicated, the term "cis-trans isomers" or "geometrical isomers" refers to isomers that result from the restricted rotation around double bonds or single bonds of cyclic carbon atoms.

Unless otherwise indicated, the term "diastereomers" refers to stereoisomers that have two or more chiral centers and exhibit non-mirror-image relationships between molecules.

Unless otherwise indicated, "(+)" represents dextro, "(-)" represents levo, "(±)" represents racemic.

Unless otherwise indicated, a wedge solid line bond (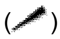) and a wedge dotted line bond (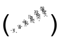) are used to denote the absolute configuration of a stereocenter, a straight solid line bond (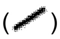) and a straight dotted line bond (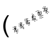) are used to denote the relative configuration of a stereocenter, a wavy line (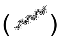) is used to denote a wedge solid line bond (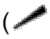) or a wedge dotted line bond (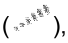), or a wavy line (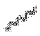) is used to denote a straight solid line bond (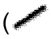) or a straight dotted line bond (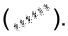).

Unless otherwise indicated, the term "enrich in one isomer", "isomer-enriched", "enrich in one enantiomer" or "enantiomer-enriched" means that the content of the one isomer or enantiomer therein is less than 100% but greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise indicated, the term "isomer excess" or "enantiomer excess" refers to the difference between the relative percentages of the two isomers or the two enantiomers. For example, the content of one isomer or enantiomer is 90%, and the content of the other isomer or enantiomer is 10%, then the isomer or enantiomer is 80% excess (ee value).

Optically active (*R*)- and (*S*)-isomers as well as *D* and *L*-isomers may be prepared by chiral synthesis or using chiral reagents or other conventional techniques. To obtain one enantiomer of a compound of the present application, it may be prepared through asymmetric synthesis or derivatization with a chiral auxiliary agent. The resulting diastereomeric mixture is then separated and the auxiliary group is cleaved to provide the pure desired enantiomer. Alternatively, when the molecule contains a basic functional group (such as an amino group) or an acidic functional group (such as a carboxyl group), salts of diastereomers may be formed by reacting with an appropriate optically active acid or base. The diastereomers may then be resolved through conventional methods known in the art, followed by recovery of the

pure enantiomers. In addition, the separation of enantiomers and diastereomers is generally achieved by using chromatography in which chiral stationary phases are employed, optionally in combination with chemical derivatization (for example, generation of carbamates from amines).

The compound of the present application may contain an unnatural proportion of atomic isotope on one or more of the atoms that constitute the compound. For example, the compound may be labeled with a radioactive isotope, e.g., tritium ($^3H$), iodine-125 ($^{125}I$) or C-14 ($^{14}C$). Further for example, hydrogen may be replaced by deuterium to form a deuterated drug. The bond between deuterium and carbon is stronger than that between ordinary hydrogen and carbon, and compared with a non-deuterated drug, the deuterated drug has the advantages of reduced toxic and side effect, increased drug stability, enhanced efficacy, and extended biological half-life of drugs. All variations in isotopic composition of the compound of the present application, whether radioactive or not, are included within the scope of the present application.

The term "optional" or "optionally" means that the event or circumstance subsequently described may, but not necessarily, occur, and that the term includes situations in which the event or circumstance occurs as well as situations in which the event or circumstance does not occur.

The term "substituted" means that any one or more hydrogen atoms on a particular atom are replaced by a substituent, which may include deuterium and hydrogen variants, provided that the valence of the particular atom is normal and the substituted compound is stable. When the substituent is oxygen (i.e., =O), it means that two hydrogen atoms are replaced. Oxygen substitution does not occur on an aromatic group. The term "optionally substituted" means substituted or unsubstituted and, unless otherwise specified, the type and number of substituent may be arbitrary on a chemically achievable basis.

When any variable (e.g., R) occurs more than once in the composition or structure of a compound, its definition in each case is independent. Therefore, for example, if a group is substituted with 0-2 R, the group may optionally be substituted with up to two R, with independent options for R in each case. In addition, the combination of substituent and/or variant thereof is permitted only if such combination results in a stable compound.

When the number of a linking group is 0, such as $-(CRR)_0-$, it means that the linking group is a single bond.

When the number of a substituent is 0, it means that the substituent is absent. For example, $-A-(R)_0$ means that the structure is actually -A.

When a substituent is vacant, it means that the substituent is absent. For example, when X in A-X is vacant, it means that the structure is actually A.

When one of the variables is selected from a single bond, it means that the two groups attached by the single bond are directly connected. For example, when L in A-L-Z denotes a single bond, it means that the structure is actually A-Z.

When the bond of a substituent can be cross-linked to more than two atoms on a ring, the substituent can be bonded to any atom on the ring. For example, the structural unit

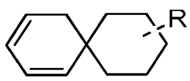

or

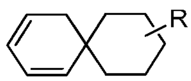

means that its substituent R can be substituted at any position on the cyclohexyl or cyclohexadiene. When a substituent listed does not indicate the atom through which it is attached to the substituted group, the substituent may be bonded through any of its atoms. For example, a pyridyl, as a substituent, may be attached to the substituted group through any carbon atom on the pyridine ring.

When a linking group listed does not indicate its linking direction, its linking direction is arbitrary. For example, the linking group L in

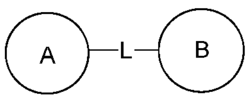

is -M-W-, and -M-W- may either link the ring A and ring B in the same direction as the reading order from left to right to form

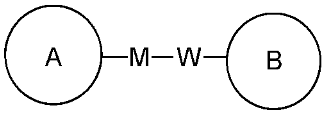

,

or link the ring A and ring B in the opposite direction to the reading order from left to right to form

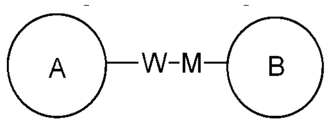

. Combinations of the linking groups, substituents and/or variants thereof are permitted only if such combinations result in stable compounds.

Unless otherwise specified, when a group has one or more linkable sites, any one or more of the sites of that group may be linked to other groups through chemical bonds. If the chemical bond is connected in a non-directional manner and H atoms are present at the connectable site, the number of H atoms at the site is correspondingly reduced with the number of chemical bonds attached when the chemical bonds are attached, forming a group of the corresponding valence. The chemical bonds connecting this site to other groups may be denoteed by a straight solid line bond

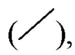

a straight dotted line bonds

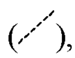

or a wavy line

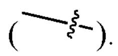

For example, the straight solid line bond in $-OCH_3$ indicates that the group is connected to other groups through the oxygen atom in that group; the straight dotted line bond in

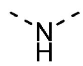

indicates that the group is connected to other groups at the two ends of the nitrogen atom in that group; and the wavy line in

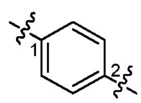

indicates that the phenyl group is connected to other groups through the carbon atoms at positions 1 and 2 of the phenyl group.

Unless otherwise specified, the number of atoms on a ring is usually defined as the number of members of the ring. For example, a "5-7-membered ring" is a "ring" with 5-7 atoms arranged around it.

Unless otherwise specified, $C_{n-n+m}$ or $C_n$-$C_{n+m}$ includes any one specific instance of n to n+m carbons, e.g., $C_{1-12}$ includes $C_1$, $C_2$, $C_3$, $C_4$, $C_5$, $C_6$, $C_7$, $C_8$, $C_9$, $C_{10}$, $C_{11}$, and $C_{12}$, and also includes any range from n to n+m, e.g., $C_{1-12}$ includes $C_{1-3}$, $C_{1-6}$, $C_{1-9}$, $C_{3-6}$, $C_{3-9}$, $C_{3-12}$, $C_{6-9}$, $C_{6-12}$, and $C_{9-12}$, etc. Similarly, n to n+m-membered means that the number of atoms on the ring is n to n+m, e.g., 3-12-membered ring include 3-membered ring, 4-membered ring, 5-membered ring, 6-membered ring, 7-membered ring, 8-membered ring, 9-membered ring, 10-membered ring, 11-membered ring, and 12-membered ring, and also include any range from n to n+m, e.g., 3-12-membered ring include 3-6-membered ring, 3-9-membered ring, 5-6-membered ring, 5-7-membered ring, 6-7-membered ring, 6-8-membered ring, and 6-10-membered ring, etc.

Unless otherwise specified, the term "$C_{1-8}$ alkyl" is used to denote a linear or branched saturated hydrocarbon group composed of 1 to 8 carbon atoms. The $C_{1-8}$ alkyl may include $C_{1-6}$, $C_{1-5}$, $C_{1-4}$, $C_{1-3}$, $C_{1-2}$, $C_{2-6}$, $C_{2-4}$, $C_8$, $C_7$, $C_6$, and $C_5$ alkyl, etc., and it can be monovalent (e.g., methyl), divalent (e.g., methylene), or multivalent (e.g., methylidyne). Examples of $C_{1-8}$ alkyl may include, but not limited to, methyl (Me), ethyl (Et), propyl (including n-propyl and isopropyl), butyl (including n-butyl, isobutyl, s-butyl, and t-butyl), pentyl (including n-pentyl, isopentyl, and neopentyl), hexyl, heptyl,

octyl, etc.

Unless otherwise specified, the term "$C_{1-4}$ alkyl" is used to denote a linear or branched saturated hydrocarbon group composed of 1 to 4 carbon atoms. The $C_{1-4}$ alkyl may include $C_{1-2}$, $C_{1-3}$, and $C_{2-3}$ alkyl, etc., and it can be monovalent (e.g., methyl), divalent (e.g., methylene), or multivalent (e.g., methylidyne). Examples of $C_{1-4}$ alkyl may include, but not limited to, methyl (Me), ethyl (Et), propyl (including *n*-propyl and isopropyl), butyl (including *n*-butyl, isobutyl, *s*-butyl, and *t*-butyl), etc.

Unless otherwise specified, the term "$C_{1-3}$ alkyl" is used to denote a linear or branched saturated hydrocarbon group composed of 1 to 3 carbon atoms. The $C_{1-3}$ alkyl may include $C_{1-2}$ and $C_{2-3}$ alkyl, etc., and it can be monovalent (e.g., methyl), divalent (e.g., methylene), or multivalent (e.g., methylidyne). Examples of $C_{1-3}$ alkyl may include, but not limited to, methyl (Me), ethyl (Et), propyl (including *n*-propyl and isopropyl), etc.

Unless otherwise specified, the "$C_{3-10}$ cycloalkyl" is used to denote a saturated cyclic hydrocarbon group composed of 3 to 10 carbon atoms, which includes monocyclic, bicyclic and tricyclic systems, where the bicyclic and tricyclic systems include spiro ring, parallel ring and bridged ring. The $C_{3-10}$ cycloalkyl may include $C_{3-8}$, $C_{3-6}$, $C_{3-5}$, $C_{4-10}$, $C_{4-8}$, $C_{4-6}$, $C_{4-5}$, $C_{5-8}$ or $C_{5-6}$ cycloalkyl, etc., and it can be monovalent, divalent, or multivalent. Examples of $C_{3-10}$ cycloalkyl may include, but not limited to, cyclopropyl, cyclobutyl, cyclopentyl, cyclohexyl, cycloheptyl, norbornyl, [2.2.2] bicyclooctane, [4.4.0]bicyclodecane, etc.

Unless otherwise specified, the "$C_{3-8}$ cycloalkyl" is used to denote a saturated cyclic hydrocarbon group composed of 3 to 8 carbon atoms, which includes monocyclic, bicyclic and tricyclic systems, wherein the bicyclic and tricyclic systems include spiro ring, parallel ring and bridged ring. The $C_{3-8}$ cycloalkyl may include $C_{3-8}$, $C_{3-6}$, $C_{3-5}$, $C_{4-8}$, $C_{4-6}$, $C_{4-5}$, $C_{5-8}$ or $C_{5-6}$ cycloalkyl, etc., and it can be monovalent, divalent, or multivalent. Examples of $C_{3-8}$ cycloalkyl may include, but not limited to, cyclopropyl, cyclobutyl, cyclopentyl, cyclohexyl, cycloheptyl, spiro[2.4]cyclohexane, etc.

Unless otherwise specified, "$C_{3-6}$ cycloalkyl" is used to denote a saturated cyclic hydrocarbon group composed of 3 to 6 carbon atoms, which includes monocyclic, bicyclic and tricyclic systems, where the bicyclic and tricyclic systems include spiro rings parallel ring and bridged ring. The $C_{3-6}$ cycloalkyl may include $C_{3-4}$, $C_{3-5}$, $C_{4-5}$, $C_{5-8}$ or $C_{5-6}$ cycloalkyl, etc., and it can be monovalent, divalent, or multivalent. Examples of $C_{3-6}$ cycloalkyl may include, but not limited to, cyclopropyl, cyclobutyl, cyclopentyl, cyclohexyl, etc.

Unless otherwise specified, the term "3-10-membered heterocycloalkyl", by itself or in combination with other terms, denotes a saturated cyclic group composed of 3 to 10 ring atoms, respectively, 1, 2, 3 or 4 of which are independently selected from O, S, N, P, and Se heteroatoms, the rest being carbon atoms, where nitrogen atoms are optionally quaternized, and nitrogen, sulphur, and phosphorus heteroatoms may be optionally oxidized (i.e., NO, $S(O)_p$, and $P(O)_p$, where p is 1 or 2). The heterocycloalkyl includes monocyclic, bicyclic and tricyclic systems, where the bicyclic and tricyclic systems include spiro ring, parallel ring and bridged ring. In addition, with regard to the "3-10-membered heterocycloalkyl", the heteroatom may occupy a position where the heterocycloalkyl is attached to the rest of the molecule. The 3-10-membered heterocycloalkyl includes 3-9-membered, 3-8-membered, 3-6-membered, 5-9-membered, 5-membered, 6-membered, 7-membered, 8-membered, and 9-membered heterocycloalkyl, etc. Examples of 3-10-membered heterocycloalkyl may include, but not limited to, azetidinyl, oxetanyl, thietanyl, pyrrolidinyl, pyrazolidinyl, imidazolidinyl, tetrahydrothienyl (including tetrahydrothien-2-yl and tetrahydrothien-3-yl, etc.), tetrahydrofuranyl (including tetrahydrofuran-2-yl, etc.), tetrahydropyranyl, piperidinyl (including 1-piperidinyl, 2-piperidinyl, and 3-piperidinyl, etc.), piperazinyl (including 1-piperazinyl and 2-piperazinyl, etc.), morpholinyl (including 3-morpholinyl and 4-morpholinyl, etc.), dioxanyl, dithianyl, isoxazolidinyl, isothiazolidinyl, 1,2-oxazinyl, 1,2-thiazinyl, hexahydropyridazinyl, homopiperazinyl, homopiperidinyl, or dioxepanyl, etc.

Unless otherwise specified, the term "5-membered heterocycloalkyl", by itself or in combination with other terms, denotes a saturated cyclic group composed of 5 ring atoms, respectively, 1, 2, or 3 of which are independently selected from O, S, N, P, and Se heteroatoms, the rest being carbon atoms, where nitrogen atoms are optionally quaternized, and nitrogen, sulphur, and phosphorus heteroatoms may be optionally oxidized (i.e., NO, $S(O)_p$, and $P(O)_p$, where p is 1 or 2). Examples of 5-membered heterocycloalkyl may include, but not limited to, pyrrolidinyl, pyrazolidinyl, imidazolidinyl, tetrahydrothienyl (including tetrahydrothien-2-yl and tetrahydrothien-3-yl, etc.), tetrahydrofuranyl (including tetrahydrofuran-2-yl, etc.), tetrahydropyranyl, etc.

The compound of the present application may be prepared by a variety of synthetic methods well known to those skilled in the art, including the specific embodiments listed below, the embodiments resulted from the combination with other chemical synthetic methods, and equivalent alternatives well known to those skilled in the art. The preferred embodiments may include, but not limited to, the examples of the present application.

The structure of the compound of the present application may be determined by conventional methods well known to those skilled in the art. If the present application involves the absolute configuration of the compound, the absolute configuration may be confirmed using routine techniques in the art. For example, single crystal X-ray diffraction (SXRD) may be used, in which a Bruker D8 venture diffractometer is used to collect diffraction intensity data of the cultured single crystal with CuK$\alpha$ radiation as the light source and in a scanning mode of $\varphi/\omega$ scanning, and after collecting relevant data, the crystal structure may be further resolved by using a direct method (Shelxs97), thus confirming the

absolute configuration.

The solvents used herein are commercially available.

The following abbreviations are used herein:

ACN represents acetonitrile; Boc represents t-butoxycarbonyl; Bn represents benzyl; DCM represents dichloromethane; DMSO represents dimethylsulfoxide; °C represents degree Celsius; hr represents hour; $LiBH_4$ represents sodium borohydride; THF represents tetrahydrofuran; Ts represents tosyl; Ac represents acetyl; Me represents methyl; and Et represents ethyl.

Compounds are named according to conventional nomenclature in the field or using ChemDraw® software, and commercially available compounds are named in suppliers' catalogs.

## DETAILED DESCRIPTION

The present application will be described in detail through embodiments below, but it does not imply that any unfavorable limitation is imposed to the present application. Although the present application has been described in detail herein where specific embodiments are also disclosed, it is apparent to those skilled in the art that various change and modification may be made to the specific embodiments of the present application without departing from the essence and scope of the present application.

### Embodiment 1

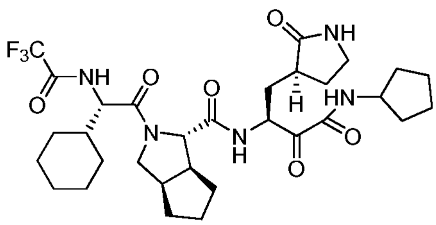

1

Synthetic route:

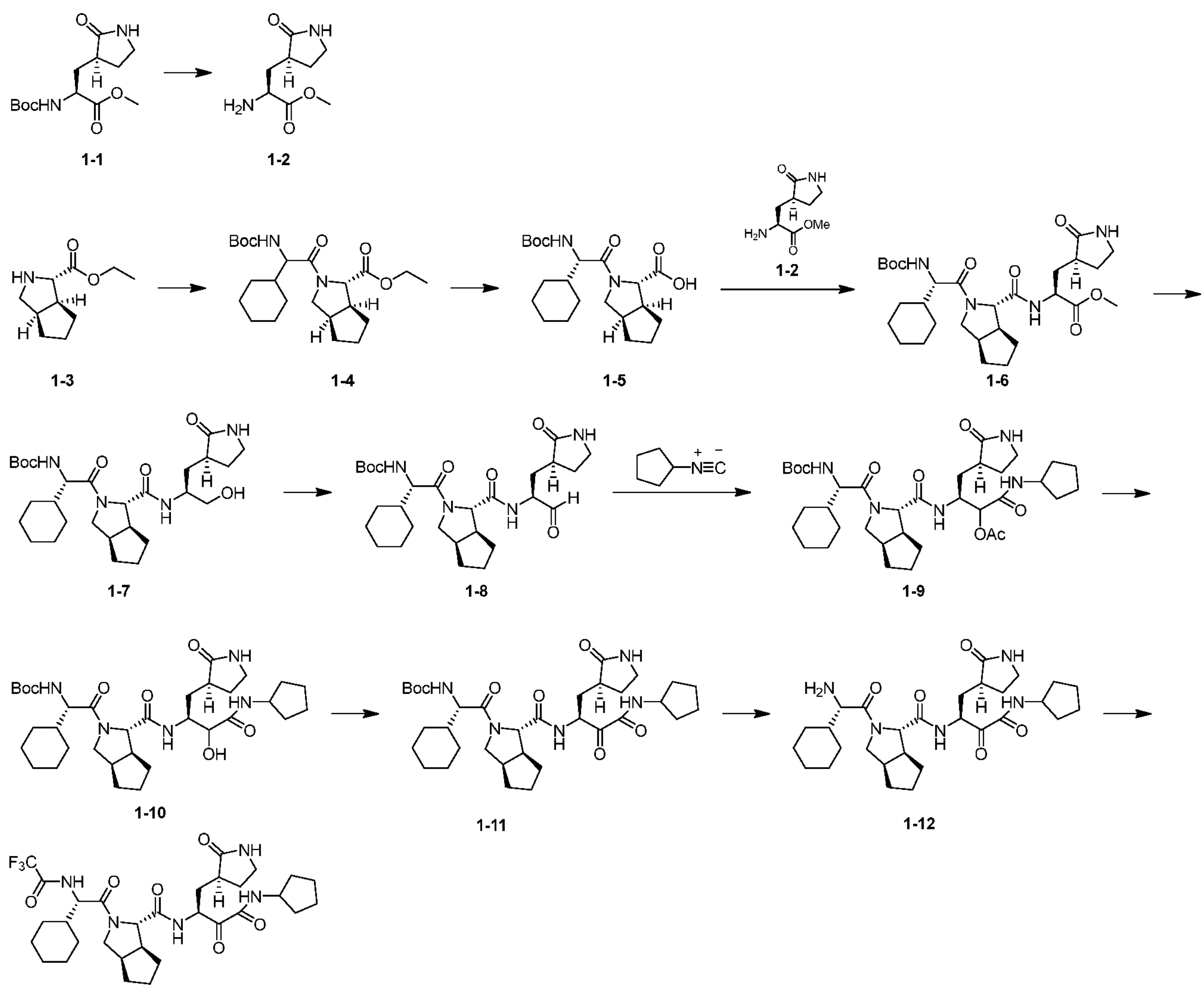

1-1 1-2

1-3 1-4 1-5 1-2 1-6

1-7 1-8 1-9

1-10 1-11 1-12

1

Step 1: Synthesis of hydrochloride of Compound **1-2**

Compound **1-1** (500 mg, 1.75 mmol) was dissolved in ethyl acetate (5 mL), into which was added a solution of hydrogen chloride in ethyl acetate (10 mL, 4 N) to react while stirring at 20°C for 2 hr. The reaction solution was concentrated under reduced pressure, without purification, to obtain a hydrochloride of Compound **1-2.** $^1$H NMR (400 MHz, $CD_3OD$) $\delta$ = 4.28 - 4.20 (m, 1H), 3.91 - 3.81 (m, 3H), 3.45 - 3.35 (m, 2H), 2.86 - 2.74 (m, 1H), 2.48 - 2.36 (m, 1H), 2.29 - 2.19 (m, 1H), 2.02 - 1.94 (m, 1H), 1.93 - 1.80 (m, 1H).

Step 2: Synthesis of Compound **1-4**

A compound Boc-L-cyclohexylglycine (1 g, 3.89 mmol) was added into N,N-dimethylformamide (10 mL), into which was added 2-(7-azabenzotriazole)-*N,N,N,N*-tetramethyluronium hexafluophosphate (1.77 g, 4.66 mmol) to react while stirring for 0.5 hr. Diisopropylethylamine (1.26 g, 9.72 mmol) and a hydrochloride of Compound **1-3** (1.02 g, 4.66 mmol) were added to react while stirring at 20°C for 16 hr. Methyl tert-butyl ether (50 mL) was added into the reaction solution, which was washed with water (20 mL), 3% citric acid (20 mL×2), and saturated sodium chloride solution (20 mL). The organic layer was dried over anhydrous sodium sulfate, then filtered, concentrated under reduced pressure, and purified by silica gel column chromatography (petroleum ether: ethyl acetate = 3:1) to obtain Compound **1-4.** $^1$H NMR (400MHz, $CDCl_3$) $\delta$ = 5.22 - 5.11 (m, 1H), 4.36 (d, *J*=3.9 Hz, 1H), 4.27 (dd, *J*=6.9, 9.3 Hz, 1H), 4.21 - 4.12 (m, 2H), 3.83 (dd, *J*=7.8, 10.4 Hz, 1H), 3.70 (br dd, *J*=3.6, 10.4 Hz, 1H), 2.81 - 2.61 (m, 2H), 1.82 - 1.70 (m, 6H), 1.68 - 1.61 (m, 4H), 1.56 - 1.48 (m, 2H), 1.46 - 1.38 (m, 9H), 1.29 - 1.22 (m, 4H), 1.21 - 0.98 (m, 4H).

Step 3: Synthesis of Compound **1-5**

Compound **1-4** (1.41 g, 3.34 mmol) was added into tetrahydrofuran (14 mL), into which was added a solution of lithium hydroxide monohydrate $LiOH \cdot H_2O$ (280.03 mg, 6.67 mmol) in water (5 mL) to react while stirring at 20°C for 16 hr. The crude product was neutralized with 3% citric acid solution (50 mL) and extracted with ethyl acetate (50 mL). The organic phase was washed with saturated sodium chloride solution (30 mL). The organic layer was dried over anhydrous sodium sulfate, then filtered, and concentrated under reduced pressure to obtain Compound **1-5** without purification. $^1H$ NMR (400MHz, DMSO-$d_6$) $\delta$ = 12.58 - 12.23 (m, 1H), 6.92 - 6.82 (m, 1H), 4.11 - 3.94 (m, 2H), 3.82 - 3.76 (m, 1H), 3.72 - 3.62 (m, 1H), 2.73 - 2.64 (m, 1H), 2.62 - 2.55 (m, 1H), 1.92 - 1.42 (m, 12H), 1.40 - 1.32 (m, 9H), 1.18 - 1.06 (m, 3H), 1.00 - 0.81 (m, 2H).

Step 4: Synthesis of Compound **1-6**

Compound **1-5** (650 mg, 1.65 mmol) was added into 2-butanone (7 mL), into which were added 1-hydroxybenzotriazole (222.63 mg, 1.65 mmol), 1-(3-dimethylaminopropyl)-3-ethylcarbodiimide hydrochloride (379.03 mg, 1.98 mmol), and diisopropylethylamine (638.84 mg, 4.94 mmol) to react while stirring at 20°C for 0.5 hr. The hydrochloride of Compound **1-2** (366.88 mg, 1.65 mmol) was then added to react while stirring at 20°C for 16 hr. Water (20 mL) was added into the reaction solution, which was extracted with dichloromethane: methanol (30 mL×2, 10:1). The organic phase was combined, washed with 3% citric acid (20 mL×2) and saturated sodium chloride solution (20 mL). The organic layer was dried over anhydrous sodium sulfate, then filtered, concentrated under reduced pressure, and purified by silica gel column chromatography (dichloromethane: methanol = 20:1) to obtain Compound 1-6. $^1H$ NMR (400 MHz, $CDCl_3$) $\delta$ = 7.49 - 7.42 (m, 1H), 6.23 - 6.05 (m, 1H), 5.28 - 5.17 (m, 1H), 4.64 - 4.51 (m, 1H), 4.43 - 4.24 (m, 2H), 3.92 - 3.81 (m, 1H), 3.78 - 3.70 (m, 3H), 3.39 - 3.27 (m, 2H), 2.94 - 2.75 (m, 2H), 2.57 - 2.36 (m, 2H), 2.24 - 2.07 (m, 1H), 1.94 - 1.50 (m, 14H), 1.49 - 1.41 (m, 9H), 1.27 - 0.95 (m, 6H).

Step 5: Synthesis of Compound **1-7**

Compound 1-6 (3.10 g, 5.51 mmol) was dissolved in tetrahydrofuran (31 mL), into which was added lithium borohydride (240.02 mg, 11.02 mmol) and warmed slowly to 20°C to react for 2 hr. Water (10 mL) and ethyl acetate (20 mL) were added into the reaction solution and stirred for 10 min. A white solid was precipitated and filtered to obtain a filter cake, which was the crude product of the target product **1-7.** [M+1]+ = 535.4.

Step 6: Synthesis of Compound **1-8**

Compound **1-7** (0.5 g, 935.13 μmol) was dissolved in dichloromethane (10 mL), into which was then added Dess-Martin periodinane (594.94 mg, 1.40 mmol) to react while stirring at 25°C for 16 hr. Saturated sodium thiosulfate (15 mL) and saturated sodium bicarbonate solution (15 mL) were added into the reaction system and stirred for 10 min. The reaction solution was extracted with dichloromethane (50 mL×2), and the organic phase was washed with saturated brine (15 mL), dried over anhydrous sodium sulfate, filtered, and concentrated to obtain a crude product of Compound **1-8.** [M+1]+ = 533.4.

Step 7: Synthesis of Compound **1-9**

Compound **1-8** (436 mg, 818.52 μmol) was dissolved in dichloromethane (5 mL), into which were added glacial acetic acid (58.98 mg, 982.22 mmol) and cyclopentyl isocyanide (94.44 mg, 982.22 μmol) to react while stirring at 25°C for 2 hr. Saturated ammonium chloride solution (10 mL) was added into the reaction system and stirred for 10 min. The reaction solution was extracted with dichloromethane (20 mL), and the organic phase was washed with water (10 mL), then dried over anhydrous sodium sulfate, filtered, concentrated under reduced pressure, and purified by silica gel column chromatography (dichloromethane: methanol = 10:1) to obtain Compound 1-9. [M+1]+ = 688.4.

Step 8: Synthesis of Compound **1-10**

Compound **1-9** (190 mg, 276.22 μmol) was dissolved in methanol (3 mL), into which was then added a solution of potassium carbonate (95.44 mg, 690.54 μmol) in water (2 mL) to react while stirring at 20°C for 16 hr. 3% citric acid (20 mL) was added into the reaction system. The reaction solution was extracted three times with dichloromethane (40 mL), and the organic phase was washed with saturated sodium chloride solution (30 mL), dried over anhydrous sodium sulfate, filtered, and concentrated under reduced pressure to obtain a crude product of **1-10.** $[M+1]^+$ = 646.5.

Step 9: Synthesis of Compound **1-11**

Compound **1-10** (238.00 mg, 368.52 μmol) was dissolved in dichloromethane (24 mL), into which was then added Dess-Martin periodinane (203.19 mg, 479.08 μmol) to react while stirring at 20°C for 18 hr. Sodium thiosulfate (15 mL) and sodium bicarbonate solution (15 mL) were added into the reaction system and stirred for 10 min. The reaction solution was extracted with dichloromethane (50 mL×2), and the organic phase was washed with saturated brine (15 mL), dried over anhydrous sodium sulfate, filtered, concentrated, and purified by silica gel column chromatography (dichloromethane: methanol = 20:1) to obtain a product **1-11.** $[M+1]^+$ = 644.5.

Step 10: Synthesis of Compound **1-12**

Compound **1-11** (125 mg, 194.16 μmol) was dissolved in tetrahydrofuran (3 mL), into which was then added hydrochloric acid in ethyl acetate (4 M, 2.91 mL) to react while stirring at 20°C for 1 hr. The reaction solution was directly rotary evaporated with an oil pump, and rotary evaporated repeatedly with a small amount of dichloromethane to obtain Compound **1-12.** $[M+1]^+$ = 544.4.

Step 11: Synthesis of Compound **1**

Compound **1-12** (125 mg, 229.91 μmol) was dissolved in tetrahydrofuran (2.5 mL), into which were added trifluoroacetic anhydride (193.15 mg, 919.63 μmol) and pyridine (127.30 mg, 1.61 mmol) at 0°C to react while stirring at 20°C for 16 hr. Water (20 mL) was added into the reaction system, which was extracted with dichloromethane (40 mL×2), and the organic phase was washed with 3% citric acid (40 mL) and saturated sodium chloride solution (40 mL×2) successively, dried over anhydrous sodium sulfate, filtered, and concentrated. The crude product was separated by preparative HPLC to obtain Compound 1. $[M+1]^+$ = 640.0; $^1$H NMR (400 MHz, $CD_3OD$) δ ppm 0.94 - 1.10 (m, 2 H), 1.13 - 1.32 (m, 3 H) 1.32 - 1.46 (m, 1 H), 1.47 - 1.57 (m, 3 H), 1.59 - 1.68 (m, 4 H), 1.69 - 1.81 (m, 6 H), 1.83 - 2.00 (m, 5 H), 2.01 - 2.17 (m, 1 H), 2.19 - 2.38 (m, 1 H), 2.49 - 2.57 (m, 1 H), 2.58 - 2.70 (m, 1 H), 2.73 - 2.89 (m, 1 H), 3.20 - 3.26 (m, 1 H), 3.37 - 3.45 (m, 1 H), 3.73 - 3.86 (m, 1 H), 3.88 - 3.97 (m, 1 H), 4.03 - 4.10 (m, 1 H), 4.11 - 4.18 (m, 1 H), 4.19 - 4.29 (m, 1 H), 4.29 - 4.37 (m, 1 H), 4.39 - 4.47 (m, 1 H), 4.57 - 4.60 (m, 2 H).

**Embodiment 2**

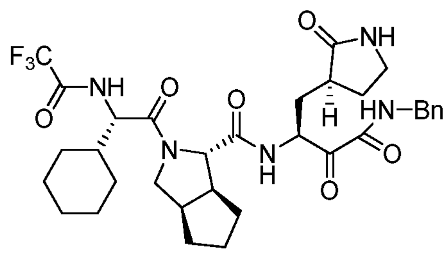

**2**

Synthetic route:

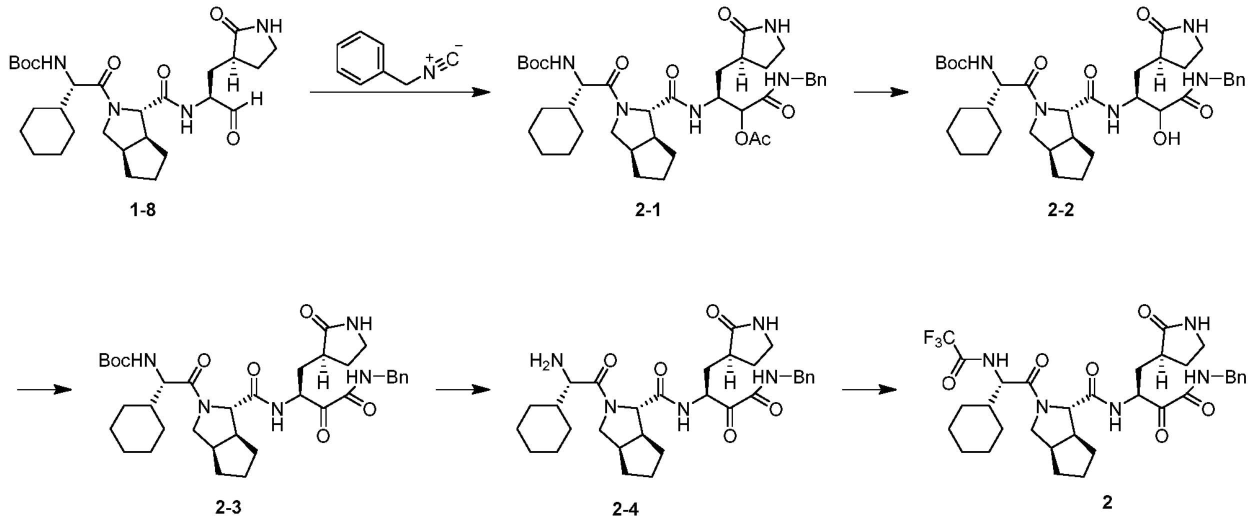

1-8 2-1 2-2

2-3 2-4 2

Step 1: Synthesis of Compound **2-1**

Compound **1-8** (630 mg, 1.18 mmol) was dissolved in dichloromethane (7 mL), into which were added glacial acetic acid (85.23 mg, 1.42 mmol) and a compound benzyl isocyanide (166.26 mg, 1.42 mmol) to react while stirring at 20°C for 16 hr. The reaction was quenched with saturated ammonium chloride solution (20 mL) and extracted with dichloromethane (40 mL×2). The organic phase was combined and washed with saturated brine (20 mL), dried over anhydrous sodium sulfate, filtered, concentrated, and purified by silica gel column chromatography (dichloromethane: methanol = 20:1) to obtain Compound **2-1.** $[M+1]^+$ = 710.4.

Step 2: Synthesis of Compound **2-2**

Compound **2-1** (519 mg, 731.12 μmol) was dissolved in anhydrous methanol (7.8 mL), into which was then added a solution of potassium carbonate (252.61 mg, 1.83 mmol) in water (5.2 mL) to react while stirring at 20°C for 16 hr. Water (10 mL) was added into the reaction solution, which was extracted twice with dichloromethane (20 mL). The organic phase was washed with 3% citric acid (10 mL) and saturated brine (10 mL), dried over anhydrous sodium sulfate, filtered, and concentrated to obtain Compound **2-2** without purification. [M+1]+ = 668.3.

Step 3: Synthesis of Compound **2-3**

Compound **2-2** (420 mg, 628.90 μmol) was dissolved in dichloromethane (4.2 mL). Dess-Martin periodinane (346.76 mg, 817.57 μmol) was then added into the reaction system to react at 20°C while stirring for 16 h. Sodium thiosulfate (15 mL) and saturated sodium bicarbonate solution (20 mL) were added into the reaction solution, which was extracted twice with dichloromethane (50 mL). The organic phase was combined, dried over anhydrous sodium sulfate, filtered, concentrated, and purified by silica gel column chromatography (dichloromethane: methanol = 20:1) to obtain Compound **2-3.** $[M+1]^+$ = 666.4.

Step 4: Synthesis of hydrochloride of Compound **2-4**

Compound **2-3** (50 mg, 75.10 μmol) was dissolved in tetrahydrofuran (0.5 mL), into which was then added a 4 M solution of hydrogen chloride in ethyl acetate (1.13 mL) to react while stirring at 20°C for 2 hr. The reaction solution was concentrated under reduced pressure and rotary evaporated repeatedly with a small amount of dichloromethane until forming a white foam, to obtain a hydrochloride of Compound **2-4.** $[M+1]^+$ = 566.4.

Step **5:** Synthesis of Compound **2**

The hydrochloride of Compound **2-4** (42.98 mg, 75.98 μmol) was dissolved in tetrahydrofuran (0.5 mL) and cooled to 0°C, into which were then added pyridine (42.07 mg, 531.83 μmol) and trifluoroacetic anhydride (63.83 mg, 303.91 μmol). The reaction solution was warmed to room temperature 20°C and stirred for 16 hr. Water (10 mL) was added into the reaction solution, which was extracted with dichloromethane (10 mL×2). The organic phase was combined and washed with 3% citric acid (10 mL) and saturated brine (10 mL), dried over anhydrous sodium sulfate, filtered, and

concentrated. The crude product was separated by preparative HPLC to obtain Compound 2. $[M+1]^+$ = 662.0.

**Embodiment 3**

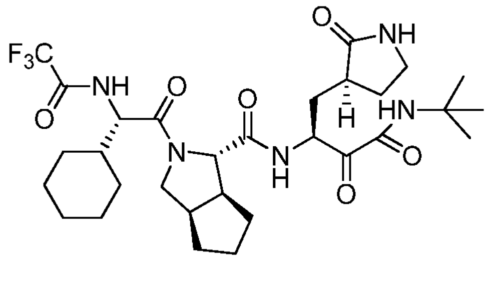

**3**

Synthetic route:

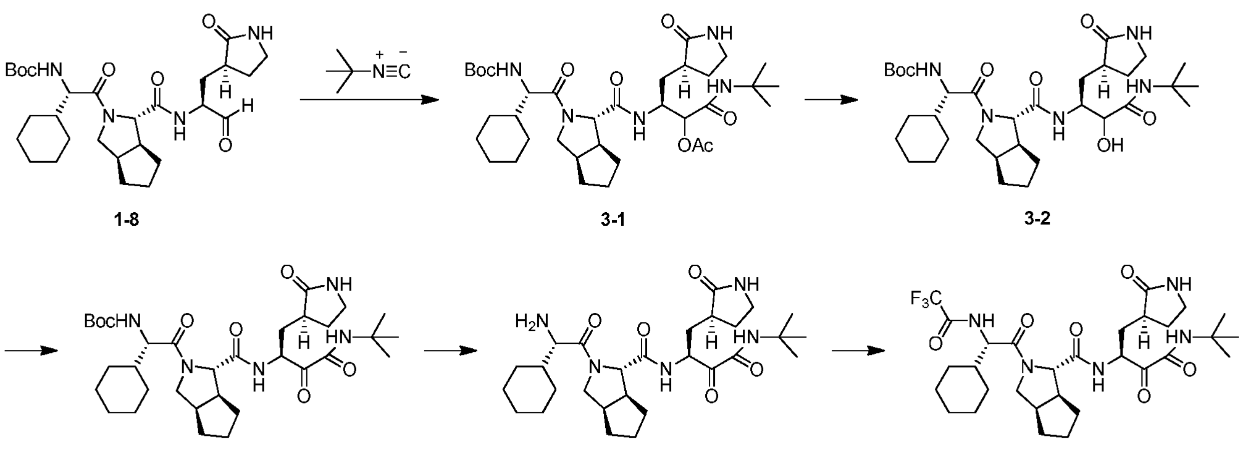

**1-8** **3-1** **3-2**

**3-3** **3-4** **3**

Step 1: Synthesis of Compound **3-1**

Compound **1-8** (223 mg, 418.65 μmol) was dissolved in dichloromethane (2.5 mL), into which were then added glacial acetic acid (30.17 mg, 502.37 μmol) and tert-butyl isocyanide (41.76 mg, 502.37 μmol) to react while stirring at 25°C for 2 hr. Saturated ammonium chloride solution (5 mL) was added into the reaction system and stirred for 10 min. The reaction solution was extracted with dichloromethane (10 mL), and the organic phase was washed with water (5 mL), dried over anhydrous sodium sulfate, filtered, concentrated under reduced pressure, and purified by silica gel column chromatography (dichloromethane: methanol = 10:1) to obtain Compound **3-1.** $[M+1]^+$ = 676.4.

Step 2: Synthesis of Compound **3-2**

Compound **3-1** (122 mg, 180.51 μmol) was dissolved in methanol (2.5 mL), into which was then added a solution of potassium carbonate (62.37 mg, 451.28 μmol) in water (1.5 mL) to react while stirring at 20°C for 16 hr. 3% citric acid (10 mL) was added into the reaction system. The reaction solution was extracted three times with dichloromethane (20 mL), and the organic phase was washed with saturated sodium chloride solution (15 mL), dried over anhydrous sodium sulfate, filtered, and concentrated under reduced pressure to obtain a crude product of Compound **3-2.** $[M+1]^+$ = 634.4.

Step 3: Synthesis of Compound **3-3**

Compound **3-2** (1.2 g, 1.89 mmol) was dissolved in dichloromethane (15 mL), into which was then added Dess-Martin periodinane (1.04 g, 2.46 mmol) to react while stirring at 20°C for 18 hr. Sodium thiosulfate (60 mL) and sodium bicarbonate solution (60 mL) were added into the reaction system and stirred for 10 min. The reaction solution was extracted with dichloromethane (120 mL×2), and the organic phase was washed with saturated brine (60 mL), dried over anhydrous sodium sulfate, filtered, concentrated, and purified by silica gel column chromatography (DCM: MeOH = 20:1) to obtain Compound **3-3.** $[M+1]^+$ = 632.5.

Step 4: Synthesis of Compound **3-4**

Compound **3-3** (380 mg, 601.46 μmol) was dissolved in tetrahydrofuran (10 mL), into which was then added hydrochloric acid in ethyl acetate (4 M, 9.02 mL) to react while stirring at 20°C for 1 hr. The reaction solution was directly dried with an oil pump and concentrated under reduced pressure repeatedly with a small amount of dichloromethane until forming white particles, to obtain Compound **3-4.** [M+1]+ = 532.4.

Step **5:** Synthesis of Compound **3**

Compound **3-4** (380 mg, 714.71 μmol) was dissolved in tetrahydrofuran (10 mL), into which were then added trifluoroacetic anhydride (395.73 mg, 5 mmol) and pyridine (600.44 mg, 2.86 mmol) to react while stirring at 20°C for 16 hr. Water (60 mL) was added into the reaction system, which was extracted with dichloromethane (120 mL×2), and the organic phase was washed with 3% citric acid (120 mL) and saturated sodium chloride solution (120 mL×2) successively, dried over anhydrous sodium sulfate, filtered, and concentrated. The crude product was separated by preparative HPLC to obtain Compound **3.** $^1H$ NMR (400 MHz, $CD_3OD$) δ ppm 0.88 - 1.33 (m, 6 H) 1.34 - 1.45 (m, 9 H) 1.47 - 2.01 (m, 15 H) 2.19 - 2.44 (m, 1 H) 2.51 - 2.90 (m, 3 H) 3.18 - 3.28 (m, 1 H) 3.69 - 4.03 (m, 2 H) 4.16 - 4.31 (m, 1 H) 4.37 - 4.48 (m, 1 H) 4.53 - 4.67 (m, 1 H). $[M+1]^+$ = 628.4.

**Embodiment 4**

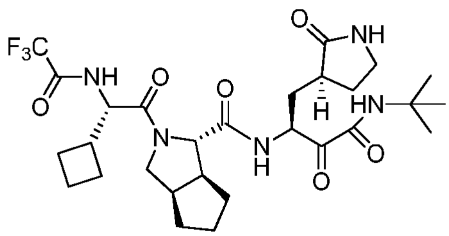

**4**

Synthetic route:

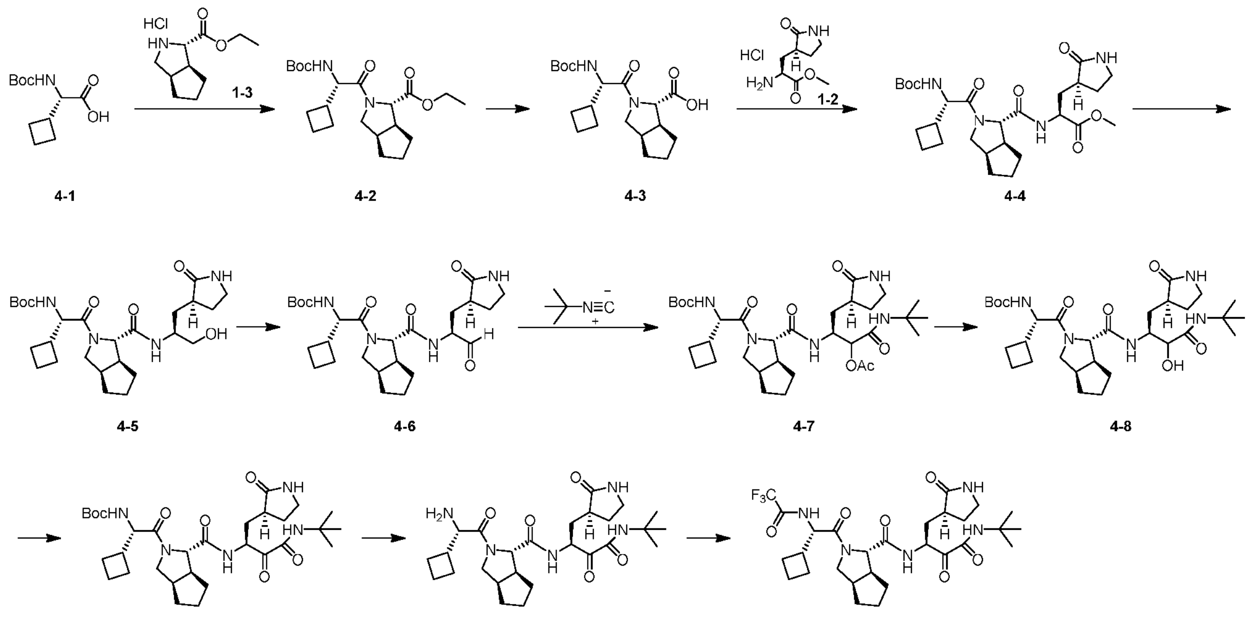

4-1 4-2 4-3 4-4

4-5 4-6 4-7 4-8

4-9 4-10 4

Step 1: Synthesis of Compound 4-2

Compound 4-1 was dissolved in N,N-dimethylformamide (5 mL), into which was added O-(7-azabenzotriazol-1-yl)-*N,N,N,N*-tetramethyluronium hexafluophosphate (1.24 g, 3.27 mmol) and stirred for 0.5 hr. Diisopropylethylamine (1.41 g, 10.90 mmol, 1.90 mL) and a hydrochloride of Compound **1-3** (527.06 mg, 2.40 mmol) were then added to react at 20°C for 2 hr. The reaction system was extracted with ethyl acetate (200 mL) and 3% citric acid solution (100 mL), and separated to obtain an organic phase, which was washed to neutral with semi-saturated brine (100 mL), dried over anhydrous sodium sulfate, filtered, concentrated, and purified by silica gel column chromatography (petroleum ether: ethyl acetate =1:0 to 10:1) to obtain Compound **4-2.** $^1$H NMR (400MHz, $CDCl_3$) δ = 5.44 - 5.24 (m, 1H), 4.44 (dd, J=6.6, 8.8 Hz, 1H), 4.36 (d, J=3.8 Hz, 1H), 4.20 - 4.14 (m, 2H), 3.85 (dd, J=7.9, 10.3 Hz, 1H), 3.66 (dd, J=3.5, 10.3 Hz, 1H), 2.75 - 2.64 (m, 2H), 1.98 - 1.59 (m, 13H), 1.50 - 1.43 (m, 9H), 1.27 - 1.25 (m, 3H).

Step 2: Synthesis of Compound **4-3**

**4-2** (0.8 g, 2.03 mmol) was dissolved in tetrahydrofuran (10 mL) and water (6 mL), into which was added lithium hydroxide monohydrate (170.19 mg, 4.06 mmol) and stirred at 20°C for 16 hr. The reaction system was extracted with dichloromethane (200 mL) and 3% citric acid (100 mL), and separated to obtain an organic phase, which was washed to neutral with saturated brine (100 mL), dried over anhydrous sodium sulfate, filtered, and concentrated to obtain Compound **4-3.** $^1$H NMR (400MHz, $CD_3OD$) δ = 4.36 (br d, *J*=8.3 Hz, 1H), 4.29 - 4.23 (m, 1H), 3.95 - 3.77 (m, 2H), 3.73 (br t, *J*=6.2 Hz, 1H), 2.76 - 2.61 (m, 2H), 2.02 - 1.57 (m, 13H), 1.44 (s, 9H).

Step 3: Synthesis of Compound **4-4**

**4-3** (0.7 g, 1.91 mmol) was dissolved in 2-butanone (15 mL), into which were added 1-hydroxybenzotriazole (258.11 mg, 1.91 mmol) and 1-(3-dimethylaminopropyl)-3-ethylcarbodiimide hydrochloride (439.43 mg, 2.29 mmol) and stirred for 0.5 hr, and subsequently added diisopropylethylamine (1.23 g, 9.55 mmol, 1.66 mL) and a hydrochloride of Compound **1-2** (467.88 mg, 2.10 mmol) to react at 20°C for 16 hr. The reaction system was extracted with 3% citric acid (100 mL) and dichloromethane (200 mL), and separated to obtain an organic phase, which was extracted with saturated brine (100 mL), and separated to obtain an organic phase, which was dried over anhydrous sodium sulfate, filtered, concentrated, and purified by silica gel column chromatography (dichloromethane: methanol = 1:0 to 10:1) to obtain Compound **4-4.** $^1$H NMR (400MHz, $CD_3OD$) δ = 8.65 (br d, *J*=8.4 Hz, 1H), 7.99 - 7.69 (m, 1H), 7.61 - 7.44 (m, 1H), 4.58 - 4.44 (m, 1H), 4.34 (d, *J*=8.0 Hz, 1H), 4.22 (d, *J*=4.0 Hz, 1H), 3.92 (dd, *J*=7.9, 10.3 Hz, 1H), 3.78 (br dd, *J*=3.6, 10.3 Hz, 1H), 3.72 (s, 3H), 3.30 - 3.26 (m, 2H), 2.89 - 2.77 (m, 1H), 2.75 - 2.50 (m, 3H), 2.36 - 2.25 (m, 1H), 2.21 - 2.08 (m, 1H), 2.05 - 1.52 (m, 14H), 1.49 - 1.39 (m, 9H).

Step 4: Synthesis of Compound **4-5**

Compound **4-4** (0.75 g, 1.40 mmol) was dissolved in tetrahydrofuran (7.5 mL) and cooled to 0°C, into which was added lithium borohydride (91.66 mg, 4.21 mmol) and warmed slowly to 20°C to react for 1 hr. Semi-saturated $NH_4Cl$ (30 mL) was added into the reaction system and stirred for 30 min to quench the reaction. The reaction solution was then extracted with dichloromethane (60 mL×2) and separated to obtain an organic phase, which was dried over anhydrous sodium sulfate, filtered, and concentrated to obtain Compound **4-5.** $^1$H NMR (400MHz, $CD_3OD$) δ = 4.35 (br d, *J*=7.9 Hz, 1H), 4.22 - 4.14 (m, 1H), 4.04 - 3.89 (m, 2H), 3.85 - 3.66 (m, 2H), 3.50 (br t, *J*=5.2 Hz, 2H), 3.29 - 3.25 (m, 1H), 2.91 - 2.78 (m, 1H), 2.74 - 2.56 (m, 3H), 2.44 (br d, *J*=7.6 Hz, 1H), 2.36 - 2.25 (m, 1H), 2.00 - 1.74 (m, 14H), 1.44 (s, 9H).

Step 5: Synthesis of Compound **4-6**

Compound **4-5** (0.55 g, 1.09 mmol) was dissolved in dichloromethane (20 mL), into which was added Dess-Martin periodinane (506.49 mg, 1.19 mmol) and stirred at 25°C for 16 hr. The reaction solution was extracted by adding saturated sodium thiosulfate solution (30 mL×2) and saturated sodium bicarbonate solution (30 mL×2) and dichloromethane (60 mL) into the reaction system, and separated to obtain an organic phase, which was dried over anhydrous sodium sulfate, filtered, and concentrated to obtain Compound **4-6.** $^1$H NMR (400MHz, $CDCl_3$) δ = 9.56 - 9.44 (m, 1H), 8.01 (br d, *J*=6.0 Hz, 1H), 6.14 - 5.97 (m, 1H), 5.50 - 5.35 (m, 1H), 4.55 - 4.29 (m, 3H), 3.94 - 3.83 (m, 1H), 3.65 (br d, *J*=7.3 Hz, 1H), 3.42 - 3.29 (m, 2H), 2.94 - 2.76 (m, 2H), 2.74 - 2.68 (m, 1H), 2.60 - 2.49 (m, 1H), 2.39 (td, *J*=3.2, 5.9 Hz, 1H), 1.97 - 1.60 (m, 15H), 1.44 (s, 8H).

Step 6: Synthesis of Compound **4-7**

Compound **4-6** (0.75 g, 1.49 mmol) was dissolved in dichloromethane (7.5 mL), into which were added glacial acetic acid (107.10 mg, 1.78 mmol) and tert-butyl isocyanide (148.27 mg, 1.78 mmol) and stirred at 20°C for 2 hr. The reaction solution was extracted by adding saturated ammonium chloride solution (30 mL) and dichloromethane (60 mL) into the reaction system, and separated to obtain an organic phase, which was dried over anhydrous sodium sulfate, filtered, and concentrated to obtain Compound **4-7.** $[M+1]^+$ = 648.2.

Step 7: Synthesis of Compound **4-8**

Compound **4-7** (0.8 g, 1.23 mmol) was dissolved in methanol (6.5 mL) and water (4 mL), into which was added potassium carbonate (426.69 mg, 3.09 mmol) and stirred at 20°C for 2 hr. The reaction system was added with saturated sodium bicarbonate solution (30 mL) and stirred for 10 min. The reaction solution was extracted with dichloromethane (60 mL), and separated to obtain an organic phase, which was dried over anhydrous sodium sulfate, filtered, concentrated, and purified by silica gel column chromatography (dichloromethane: methanol = 1:0 to 10:1) to obtain Compound **4-8.** $^1$H NMR (400MHz, $CDCl_3$) δ = 7.08 (br d, *J*=8.3 Hz, 1H), 6.87 - 6.62 (m, 1H), 6.07 - 5.94 (m, 1H), 5.98 (br s, 1H), 5.35 (br d, *J*=8.6 Hz, 1H), 4.52 - 4.30 (m, 2H), 4.28 - 3.98 (m, 3H), 3.74 - 3.57 (m, 1H), 3.33 (br d, *J*=8.3 Hz, 2H), 2.81 - 2.65 (m, 3H), 2.54 (td, *J*=8.0, 15.5 Hz, 1H), 2.41 - 2.31 (m, 1H), 2.03 - 1.69 (m, 13H), 1.63 - 1.52 (m, 2H), 1.46 - 1.41 (m, 9H), 1.38 - 1.32 (m, 9H).

Step 8: Synthesis of Compound **4-9**

Compound **4-8** (0.65 g, 1.07 mmol) was dissolved in dichloromethane (12 mL), into which was added Dess-Martin periodinane (682.67 mg, 1.61 mmol) to react at 20°C for 16 hr. The reaction solution was extracted by adding saturated sodium thiosulfate solution (30 mL×2) and saturated sodium bicarbonate solution (30 mL×2) and dichloromethane (60 mL) into the reaction system, and separated to obtain an organic phase, which was dried over anhydrous sodium sulfate, filtered, concentrated, and purified by silica gel column chromatography (dichloromethane: methanol = 1:0 to 10:1) to obtain Compound **4-9.** $[M+1]^+$ = 604.5.

Step 9: Synthesis of trifluoroacetate of Compound **4-10**

Compound **4-9** (450 mg, 745.34 μmol) was dissolved in dichloromethane (6 mL), into which was added trifluoroacetic acid (1.5 mL) to react at 20°C for 1 hr. The reaction system was concentrated under reduced pressure to obtain a trifluoroacetate of the target compound **4-10.** $^1$H NMR (400MHz, DMSO-*d6*) δ = 8.51 (d, *J*=8.4 Hz, 1H), 8.04 (br s, 3H), 7.64 (s, 1H), 5.75 (s, 1H), 5.23 - 5.05 (m, 1H), 4.29 - 4.22 (m, 1H), 4.20 - 4.10 (m, 1H), 3.79 - 3.65 (m, 1H), 3.63 - 3.55 (m, 1H), 3.15 - 3.04 (m, 1H), 2.69 (br d, *J*=7.4 Hz, 3H), 2.44 - 2.34 (m, 1H), 2.25 - 2.17 (m, 1H), 1.85 - 1.51 (m, 13H), 1.48 - 1.33 (m, 2H), 1.30 (s, 9H).

Step 10: Synthesis of Compound **4**

The trifluoroacetate of **4-10** (275 mg, 546.03 μmol) was dissolved in dichloromethane (2.7 mL), into which were added pyridine (431.91 mg, 5.46 mmol) and trifluoroacetic anhydride (286.71 mg, 1.37 mmol) at 0°C to react at 20°C for 2 hr. The reaction system was extracted with 3% citric acid solution (30 mL) and dichloromethane (60 mL*2), and separated to obtain an organic phase. The organic phase was washed to neutral with saturated brine (60 mL), dried over anhydrous sodium sulfate, filtered, concentrated, and purified by silica gel column chromatography (dichloromethane: methanol = 10:1) to obtain Compound **4.** $^1H$ NMR (400MHz, $CDCl_3$) δ = 8.09 (br d, *J*=5.9 Hz, 1H), 7.40 (br d, *J*=8.6 Hz, 1H), 6.89 - 6.71 (m, 1H), 5.92 - 5.79 (m, 1H), 5.41 - 5.20 (m, 1H), 4.90 - 4.68 (m, 1H), 4.28 (d, *J*=3.5 Hz, 1H), 4.01 - 3.82 (m, 1H), 3.61 - 3.46 (m, 1H), 3.40 - 3.29 (m, 2H), 2.86 (br d, *J*=11.0 Hz, 1H), 2.81 - 2.74 (m, 1H), 2.55 - 2.42 (m, 1H), 2.00 - 1.62 (m, 17H), 1.40 - 1.37 (m, 9H).

**Embodiment 5**

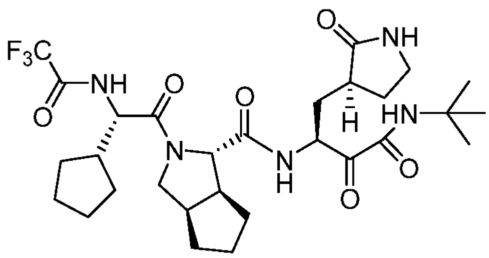

5

Synthetic route:

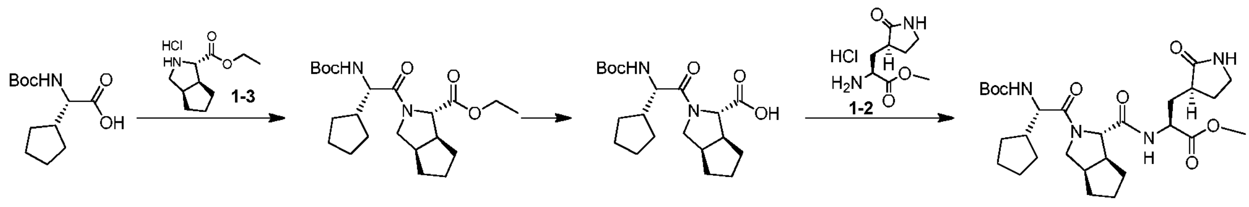


5-1 5-2 5-3 5-4

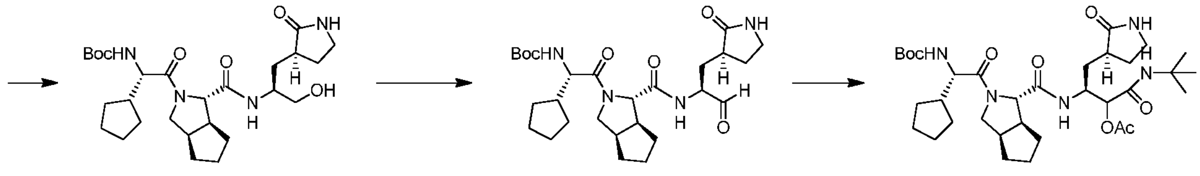

5-5 5-6 5-7

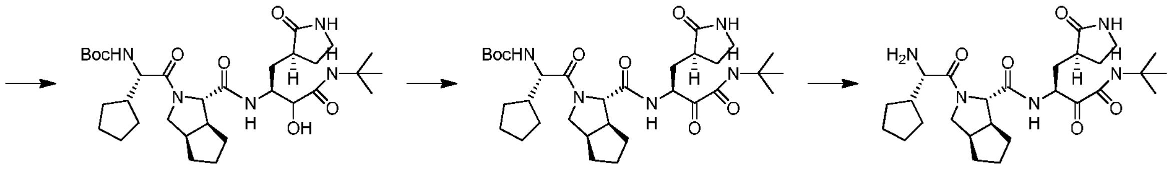

5-8 5-9 5-10

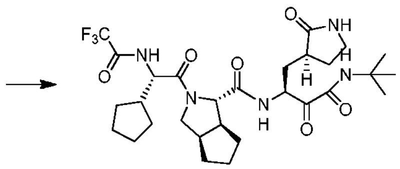

5

Step 1: Synthesis of Compound **5-2**

Compound **5-1** (0.65 g, 2.67 mmol) was dissolved in N,N-dimethylformamide (20 mL), into which was then added O-(7-azabenzotriazol-1-yl)-*N,N,N,N*-tetramethyluronium hexafluophosphate (1.22 g, 3.21 mmol) and stirred at 20°C for 0.5 hr. Diisopropylethylamine (863.20 mg, 6.68 mmol) and a hydrochloride of Compound 1-3 (704.37 mg, 3.21 mmol) were then added into the reaction system respectively to react while stirring at 20°C for 5 hr. The reaction solution was directly diluted with 3% citric acid (30 mL), extracted with ethyl acetate (60 mL), and washed with saturated brine (30 mL). The organic phase was combined and dried over anhydrous sodium sulfate, filtered, concentrated, and purified over a column. The resulting fraction was directly concentrated under reduced pressure to obtain Compound **5-2.** $[M+1]^+ = 409.2$.

Step **2:** Synthesis of Compound **5-3**

Compound **5-2** (1 g, 2.11 mmol) was dissolved in tetrahydrofuran (18 mL) and water (6 mL), into which was added lithium hydroxide monohydrate (176.83 mg, 4.21 mmol) to react while stirring at 25°C for 12 hr. The reaction solution was diluted with 3% citric acid (30 mL) and extracted with ethyl acetate (60 mL). The organic phase was combined and washed with saturated brine (30 mL), dried over anhydrous sodium sulfate, filtered, and concentrated to obtain the product Compound **5-3** without purification. $[M+1]^+ = 381.2$.

Step **3:** Synthesis of Compound **5-4**

Compound **5-3** (1 g, 2.63 mmol) was dissolved in 2-butanone (40 mL), into which were then added 1-hydroxybenzotriazole (355.13 mg, 2.63 mmol) and 1-(3-dimethylaminopropyl)-3-ethylcarbodiimide hydrochloride (604.61 mg, 3.15 mmol) to react at 25°C while stirring for 0.5 hr, and subsequently added diisopropylethylamine (1.36 g, 10.51 mmol) and a hydrochloride of Compound **1-2** (702.28 mg, 3.15 mmol) to continue reacting while stirring for 16 hr. The reaction solution was extracted with dichloromethane (30 mL), and the organic phase was washed with 3% citric acid (30 mL) and saturated brine (20 mL), dried over anhydrous sodium sulfate, filtered, concentrated, and purified over a column to

obtain Compound **5-4.** $[M+1]^+$ = 549.3.

Step **4:** Synthesis of Compound **5-5**

Compound **5-4** (1.24 g, 2.26 mmol) was dissolved in tetrahydrofuran (30 mL), into which was then added lithium borohydride (147.67 mg, 6.78 mmol) to react while stirring at 25°C for 3 hr. The reaction solution was extracted with dichloromethane (30 mL), and the organic phase was quenched with saturated ammonium chloride (30 mL), washed with saturated brine (30 mL), dried over anhydrous sodium sulfate, filtered, and concentrated to obtain Compound **5-5.** $[M+1]^+$ = 521.3.

Step **5:** Synthesis of Compound **5-6**

Compound **5-5** (0.5 g, 960.32 μmol) was dissolved in dichloromethane (20 mL), into which was then added Dess-Martin periodinane (448.04 mg, 1.06 mmol) to react while stirring at 25°C for 16 hr. The reaction was quenched with saturated sodium bicarbonate solution (20 mL) and sodium thiosulfate solution (20 mL), extracted with dichloromethane (60 mL), dried over anhydrous sodium sulfate, filtered, and concentrated to obtain Compound **5-6.** $[M+1]^+$ = 519.3.

Step **6:** Synthesis of Compound **5-7**

Compound **5-6** (0.6 g, 1.16 mmol) and glacial acetic acid (83.36 mg, 1.39 mmol) were dissolved in dichloromethane (20 mL), into which was then added tert-butyl isocyanide (115.40 mg, 1.39 mmol) to react while stirring at 25°C for 3 hr. The reaction solution was directly diluted with water (20 mL) and extracted with dichloromethane (60 mL). The organic phase was combined, dried, concentrated, and purified by column chromatography to obtain Compound **5-7.** $[M+1]^+$ = 662.4.

Step **7:** Synthesis of Compound **5-8**

Compound **5-7** (0.4 g, 604.39 μmol) was dissolved in methanol (12 mL), into which was then added a solution of potassium carbonate (208.83 mg, 1.51 mmol) in water (6 mL) to react while stirring at 25°C for 12 hr. The reaction solution was diluted with water (50 mL) and extracted with dichloromethane (60 mL). The organic phase was combined and washed with 3% citric acid (15 mL) and saturated brine (20 mL), dried over anhydrous sodium sulfate, filtered, and concentrated to obtain Compound **5-8.** $[M+1]^+$ = 620.4.

Step **8:** Synthesis of Compound **5-9**

Compound **5-8** (0.35 g, 564.71 μmol) was dissolved in dichloromethane (10 mL), into which was then added Dess-Martin periodinane (359.27 mg, 847.06 μmol) to react while stirring at 25°C for 12 hr. The reaction was quenched with saturated sodium bicarbonate solution (20 mL) and sodium thiosulfate solution (20 mL), and extracted with dichloromethane (20 mL×3). The organic phase was combined and dried over anhydrous sodium sulfate, filtered and concentrated to obtain Compound **5-9.** $[M+1]^+$ = 618.4.

Step **9:** Synthesis of Compound **5-10**

Compound **5-9** (240 mg, 388.49 μmol) was dissolved in dichloromethane (6 mL), into which was added trifluoroacetic acid (1.5 mL) to react at 20°C for 1 hr. The reaction solution was concentrated to Compound **5-10.** $[M+1]^+$ = 518.4.

Step **10:** Synthesis of Compound **5**

Compound **5-10** (180 mg, 347.72 μmol) was dissolved in dichloromethane (3 mL), into which were added pyridine (275.05 mg, 3.48 mmol) and trifluoroacetic anhydride (182.58 mg, 869.30 μmol) at 0°C to react at 20°C for 2 hr. The reaction system was extracted with dichloromethane (60 mL) and 3% citric acid solution (30 mL), and separated to obtain an organic phase, which was extracted with saturated brine (30 mL), and separated to obtain an organic phase, which was dried over anhydrous sodium sulfate, concentrated, and purified by column chromatography to obtain Compound **5.** $^1$H NMR (400 MHz, DMSO-*d6*) δ ppm 9.79 - 9.92 (m, 1 H) 8.46 (d, J=8.13 Hz, 1 H) 7.12 - 7.99 (m, 2 H) 5.95 - 6.29 (m, 1 H) 4.89 - 5.14 (m, 1 H) 4.02 - 4.38 (m, 2 H) 3.57 - 3.86 (m, 2 H) 2.87 - 3.30 (m, 3 H) 2.63 - 2.76 (m, 1 H) 2.01 - 2.42 (m, 3 H) 1.36 - 1.99 (m, 15 H) 1.26 - 1.33 (m, 9 H) 1.15 - 1.26 (m, 2 H).

**Embodiment 6**

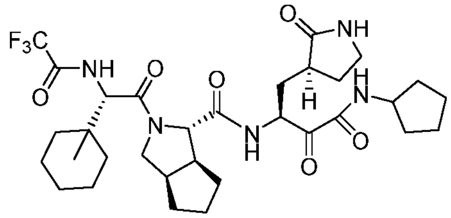

6

Synthetic route:

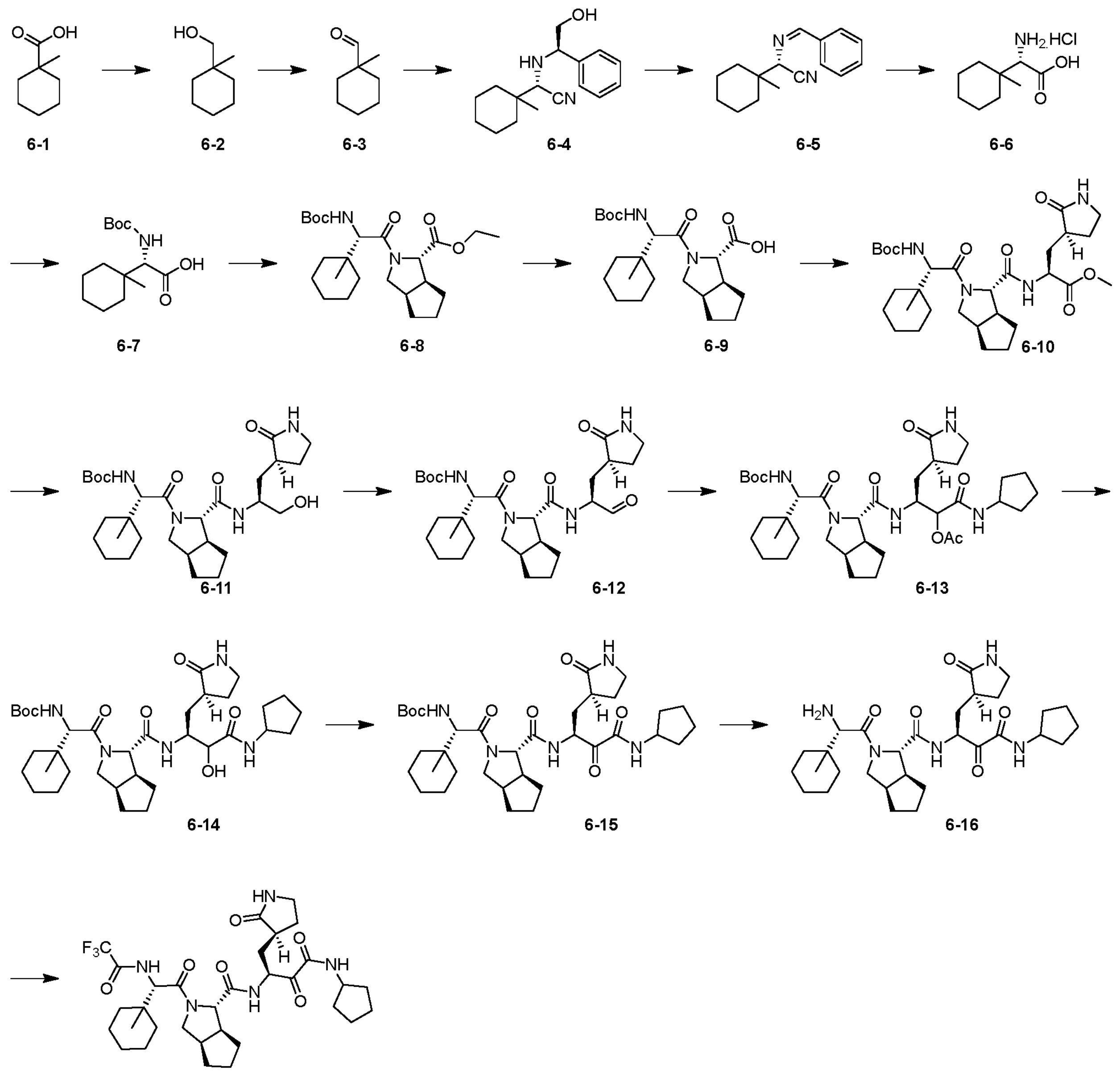

6-1 6-2 6-3 6-4 6-5 6-6

6-7 6-8 6-9 6-10

6-11 6-12 6-13

6-14 6-15 6-16

6

Step **1:** Synthesis of Compound **6-2**

Sodium borohydride (10.42 g, 275.44 mmol) was dissolved in tetrahydrofuran (300 mL) and replaced with nitrogen three times, and cooled to 0°C. Compound **6-1** (20 g, 140.65 mmol) was dissolved in tetrahydrofuran (100 mL) and dropwise added into the reaction system slowly, into which was then dropwise added boron trifluoride ethyl ether (281.30 mmol, 34.72 mL) slowly. The reaction solution was warmed to 20°C and stirred for 2 hr. Ethanol (1 L) was added and stirred for 15 min. The reaction solution was concentrated, into which was added water (100 mL), and extracted with dichloromethane (100 mL×3). The organic phase was combined, dried over anhydrous sodium sulfate, and filtered. The filtrate was concentrated under reduced pressure to obtain Compound **6-2.** $^1$H NMR (400 MHz, $CDCl_3$) δ ppm 3.6 (s, 2 H), 1.40 - 1.56 (m, 5 H), 1.23 - 1.37 (m, 5H), 0.93 (s, 3 H).

Step **2:** Synthesis of Compound **6-3**

Compound **6-2** (17.99 g, 140.32 mmol) was dissolved in dichloromethane (180 mL), into which were added pyridinium chlorochromate (45.37 g, 210.47 mmol) and silica gel (45 g, 748.59 mmol). The reaction solution was stirred at 20°C for 16 hr. The reaction solution was filtered, and the filtrate was concentrated at 20°C under reduced pressure to obtain a crude product of Compound **6-3,** which was directly used in the next reaction. $^1$H NMR (400 MHz, $CDCl_3$) δ ppm 9.37 (s, 1 H), 1.16 - 1.54 (m, 10 H), 0.93 (s, 3 H).

Step **3:** Synthesis of Compound **6-4**

Compound **6-3** (17 g, 134.71 mmol) was dissolved in chloroform (150 mL), into which was added R-phenylglycinol (18.48 g, 134.71 mmol). The reaction solution was stirred at 20°C for 2 hr and cooled to 0°C, into which was added trimethylsilyl cyanide (26.73 g, 269.42 mmol, 33.71 mL), and the reaction solution was stirred at 20°C for 16 hr. The reaction solution was concentrated under reduced pressure to dry, and the resulting crude product was purified by silica gel column chromatography (petroleum ether: ethyl acetate = 3:1) to obtain Compound **6-4.** $^1$H NMR (400 MHz, $CDCl_3$) δ ppm 7.13- 7.27 (m, 5 H), 3.89 - 3.96 (m, 1 H), 3.61 - 3.69 (m, 1 H), 3.37 - 3.47 (m, 1 H), 2.92 - 3.02 (m, 1 H), 1.03- 1.41 (m, 10 H), 0.93 (s, 3 H). $[M+1]^+$ = 273.2.

Step **4:** Synthesis of Compound **6-5**

Compound **6-4** (10 g, 36.71 mmol) was dissolved in methanol (100 mL) and dichloromethane (100 mL) and cooled to 0°C, into which was added lead tetraacetate (13.56 g, 27.53 mmol) and replaced with nitrogen three times, and the reaction solution was stirred at 0°C for 2 hr. Saturated sodium bicarbonate solution (200 mL) was added into the reaction system, which was extracted with dichloromethane (45 mL×3). The organic phase was combined, dried over anhydrous sodium sulfate, and filtered. The filtrate was concentrated under reduced pressure to obtain a crude product of Compound **6-5.** $[M+1]^+$ = 241.0.

Step **5:** Synthesis of Compound **6-6**

Compound **6-5** (5.4 g, 22.47 mmol) was dissolved in hydrochloric acid (6M, 490 mL), and the reaction solution was warmed to 100°C and stirred for 24 hr. The reaction solution was then cooled to room temperature and extracted with chloroform (300 mL×3), and the aqueous phase was concentrated under reduced pressure to obtain Compound **6-6.** $^1$H NMR (400 MHz, $CD_3OD$) δ ppm 4.46 (s, 1 H), 1.41- 1.74 (m, 10 H), 1.20 (s, 3 H).

Step **6:** Synthesis of Compound **6-7**

Compound **6-6** (3.6 g, 17.33 mmol) was dissolved in methanol (36 mL), into which were added triethylamine (52.00 mmol, 7.24 mL) and di-tert butyl dicarbonate (26.00 mmol, 5.97 mL) to react while stirring at 20°C for 4 hr. The reaction solution was adjusted to pH = 3 with aqueous citric acid solution (3%) and extracted with ethyl acetate (50 mL×3). The organic phase was combined, dried over anhydrous sodium sulfate, and filtered. The filtrate was concentrated under reduced pressure to obtain Compound **6-7.**

Step **7:** Synthesis of Compound **6-8**

Compound **6-7** (1.5 g, 5.53 mmol) and a hydrochloride of Compound **1-3** (1.46 g, 6.63 mmol) were dissolved in ethyl acetate (15 mL), into which were added *N,N*-diisopropylethylamine (2.86 g, 22.11 mmol, 3.85 mL) and n-propyl phosphate anhydride (5.28 g, 8.29 mmol, 4.93 mL, 50% solution in ethyl acetate) to react while stirring at 55°C for 16

hr. Water (30 mL) was added into the reaction solution, which was extracted with ethyl acetate (30 mL×3). The organic phase was combined, dried over anhydrous sodium sulfate, and filtered. The filtrate was concentrated under reduced pressure. The resulting crude product was purified by silica gel column chromatography (dichloromethane: methanol = 100:0 to 10: 1) to obtain Compound **6-8.** $[M+1]^+$ = 437.1.

Step **8:** Synthesis of Compound **6-9**

Compound **6-8** (1.52 g, 3.48 mmol) was dissolved in methanol (15 mL) and water (5 mL), into which was added lithium hydroxide monohydrate (166.77 mg, 6.96 mmol) to react while stirring at 20°C for 16 hr. The reaction solution was adjusted to pH = 3 with 3% aqueous citric acid solution and extracted with ethyl acetate (50 mL×3). The organic phase was combined, dried over anhydrous sodium sulfate, and filtered. The filtrate was concentrated under reduced pressure to obtain Compound **6-9.** $[M-56+H]^+$= 353.2.

Step **9:** Synthesis of Compound **6-10**

Compound **6-9** (1.42 g, 3.48 mmol) and Compound **1-1** (1.50 g, 4.18 mmol, TsOH) were dissolved in ethyl acetate (15 mL), into which were added *N,N*-diisopropylethylamine (1.80 g, 13.92 mmol, 2.42 mL) and n-propyl phosphate anhydride (5.28 g, 8.29 mmol, 4.93 mL, 50% solution in ethyl acetate) to react while stirring at 55°C for 16 hr. 30 mL of water was added into the reaction solution, which was extracted three times with ethyl acetate (30 mL). The organic phase was dried over anhydrous sodium sulfate, filtered, concentrated, and purified by silica gel column chromatography (dichloromethane: methanol = 100:0 to 10:1) to obtain Compound **6-10.** $[M+1]^+$= 577.4.

Step **10:** Synthesis of Compound **6-11**

Compound **6-10** (605 mg, 1.05 mmol) was dissolved in THF (12 mL), into which was added $LiBH_4$ (45 mg, 2.1 mmol) slowly at 0°C and warmed slowly to 20°C to react for 2 hr. Saturated ammonium chloride solution (10 mL) was slowly added into the reaction solution, which was extracted with dichloromethane (20 mL×3). The organic phase was combined, washed with saturated brine (50 mL), dried over anhydrous sodium sulfate, and filtered. The filtrate was concentrated under reduced pressure to obtain Compound **6-11.** $[M+1]^+$ = 549.1.

Step **11:** Synthesis of Compound **6-12**

Compound **6-11** (575 mg, 1.05 mmol) was dissolved in dichloromethane (10 mL), into which was added Dess-Martin periodinane (533.35 mg, 1.26 mmol) to react while stirring at 20°C for 16 hr. Saturated sodium thiosulfate solution (10 mL) and saturated sodium bicarbonate solution (10 mL) were added into the reaction solution, which was extracted with dichloromethane (20 mL×3). The organic phase was combined, dried over anhydrous sodium sulfate, and filtered. The filtrate was concentrated under reduced pressure, and the resulting crude product was purified by silica gel column chromatography (dichloromethane: methanol = 100:0 to 10:1) to obtain Compound **6-12.** $[M+1]^+$ = 547.1.

Step **12:** Synthesis of Compound **6-13**

Compound **6-12** (330 mg, 603.63 μmol) was dissolved in dichloromethane (3 mL), into which were added acetic acid (72.50 mg, 1.21 mmol, 69.04 μL) and cyclopentyl isocyanide (68.92 mg, 724.35 μmol, 80.14 μL) to react while stirring at 20°C for 2 hr. Saturated aqueous ammonium chloride solution (5 mL) was added into the reaction solution, which was extracted with dichloromethane (10 mL×3). The organic phase was combined, dried over anhydrous sodium sulfate, and filtered. The filtrate was concentrated under reduced pressure, and the resulting crude product was purified by silica gel column chromatography (dichloromethane: methanol = 100:0 to 10:1) to obtain Compound **6-13.** $[M+1]^+$ = 702.4.

Step **13:** Synthesis of Compound **6-14**

Compound **6-13** (304 mg, 433.12 μmol) was dissolved in methanol (3 mL) and water (2 mL), into which was added potassium carbonate (149.65 mg, 1.08 mmol) to react while stirring at 20°C for 4 hr. The reaction solution was adjusted to pH = 3 with 3% aqueous citric acid solution and extracted with ethyl acetate (20 mL×3). The organic phase was combined, dried over anhydrous sodium sulfate, and filtered. The filtrate was concentrated under reduced pressure to obtain a crude product of Compound **6-14,** which was directly used in the next reaction. $[M+1]^+$ = 660.2.

Step **14:** Synthesis of Compound **6-15**

Compound **6-14** (285.7 mg, 432.97 μmol) was dissolved in dichloromethane (6 mL), into which was added Dess-Martin periodinane (220.37 mg, 519.57 μmol) to react while stirring at 20°C for 16 hr. Saturated sodium thiosulfate solution (10 mL) and saturated sodium bicarbonate solution (10 mL) were added into the reaction solution, which was extracted with dichloromethane (20 mL×3). The organic phase was combined, dried over anhydrous sodium sulfate, and filtered. The filtrate was concentrated under reduced pressure, and the resulting crude product was purified by silica gel column chromatography (dichloromethane: methanol = 100:0 to 10:1) to obtain Compound **6-15.** $[M+1]^+$ = 658.4.

Step **15:** Synthesis of trifluoroacetate of Compound **6-16**

Compound **6-15** (265.8 mg, 404.05 μmol) was dissolved in dichloromethane (2 mL), into which was added trifluoroacetic acid (921.40 mg, 8.08 mmol, 598.31 μL) to react while stirring at 20°C for 2 hr. The reaction solution was concentrated under reduced pressure to obtain a trifluoroacetate of Compound **6-16,** which was directly used in the next reaction. $[M+1]^+$=558.3.

Step **16:** Synthesis of Compound **6**

The trifluoroacetate of Compound **6-16** (271 mg, 403.43 μmol) was dissolved in dichloromethane (3 mL), into which were added pyridine (223.38 mg, 2.82 mmol, 227.94 μL) and trifluoroacetic anhydride (127.10 mg, 605.14 μmol, 84.17 μL) at 0°C. The reaction solution was warmed to 20°C slowly and stirred for 2 hr. Water (10 mL) was added into the reaction solution, which was extracted with dichloromethane (20 mL×3). The organic phase was combined, washed with 3% citric acid (50 mL) and saturated brine (50 mL) respectively, dried over anhydrous sodium sulfate, and filtered. The filtrate was concentrated under reduced pressure and the resulting crude product was purified by preparative HPLC (chromatographic column: Xtimate C18 100*30 mm*3 μm; mobile phase: [A: water (formic acid) - B: acetonitrile]; acetonitrile%: 40%-80%, 8 min) to obtain Compound 6. $^1$H NMR (400 MHz, $CDCl_3$) δ ppm 7.01 - 7.27 (m, 1 H), 6.76 - 6.99 (m, 1 H), 6.05 - 6.46 (m, 1 H), 5.02 - 5.43 (m, 1 H), 4.59 - 4.75 (m, 1 H), 4.25 - 4.49 (m, 1 H), 4.09 - 4.23 (m, 1 H), 3.75-4.08 (m, 1 H), 3.59 - 3.73 (m, 1 H), 3.25 - 3.54(m, 2 H), 2.42 - 2.92 (m, 3 H), 1.82-2.37 (m, 8 H), 1.25 - 1.80 (m, 20 H), 0.90-1.20 (m, 4 H). $[M+1]^+$ = 654.4.

**Embodiment 7**

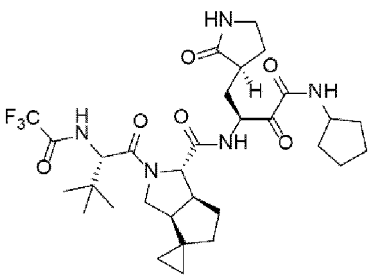

7A or 7B

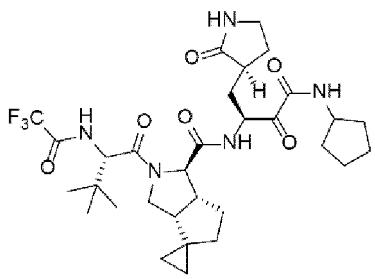

7B or 7A

Synthetic route:

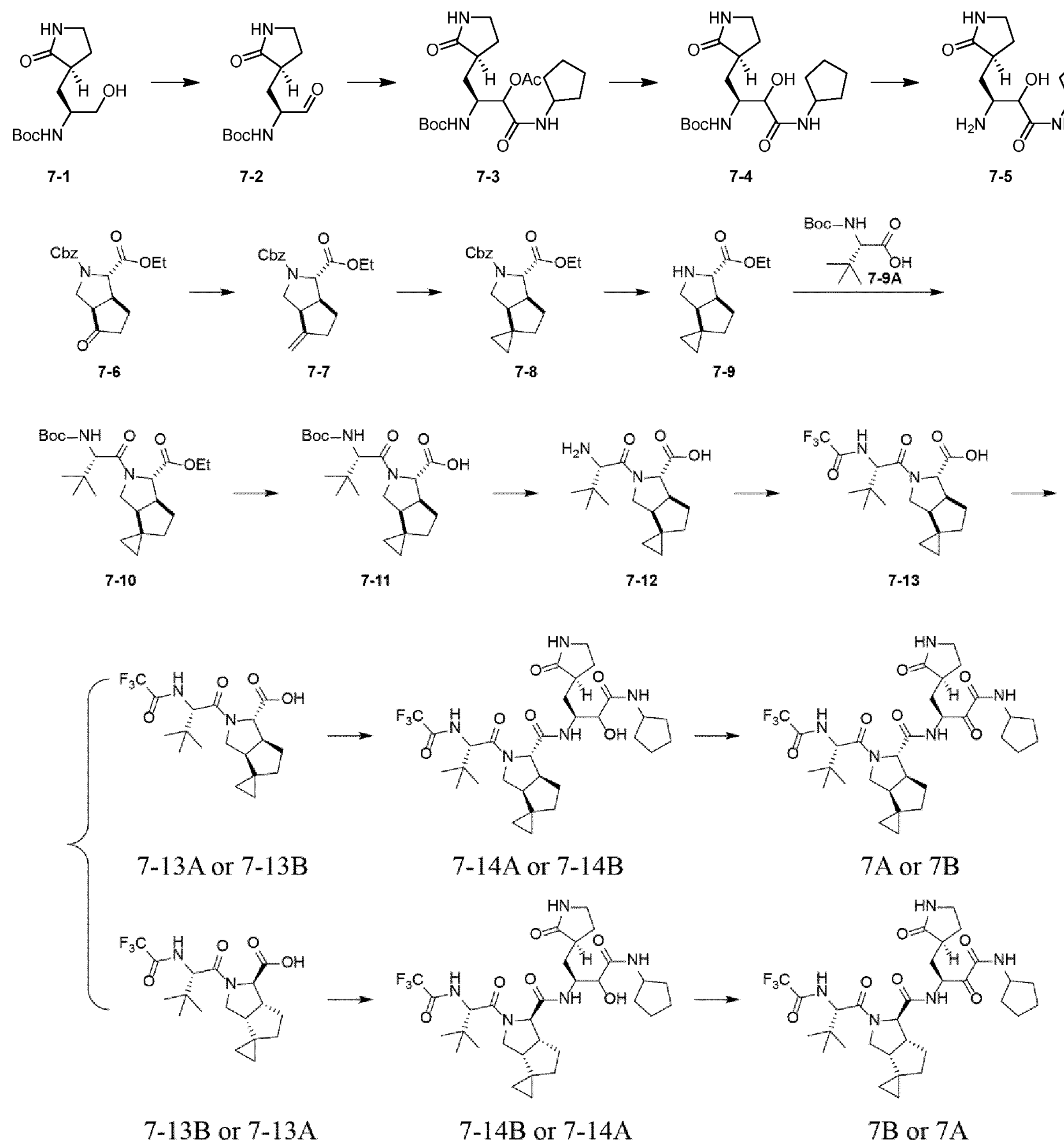


7-1 7-2 7-3 7-4 7-5

7-6 7-7 7-8 7-9

7-10 7-11 7-12 7-13

7-13A or 7-13B 7-14A or 7-14B 7A or 7B

7-13B or 7-13A 7-14B or 7-14A 7B or 7A

Step **1:** Synthesis of Compound **7-2**

Compound **7-1** (90 g, 348.41 mmol) was dissolved in dichloromethane (900 mL), into which was added Dess-Martin periodinane (162.55 g, 383.26 mmol) to react while stirring at 20°C for 2 hr. The reaction solution was quenched with saturated aqueous sodium thiosulfate solution (900 mL), adjusted to pH 7-8 with aqueous sodium carbonate solution, and separated. The aqueous phase was extracted with dichloromethane (900 mL), and the organic phase was combined and concentrated under reduced pressure to obtain a crude product of Compound **7-2,** which was directly used in the next reaction.

Step **2:** Synthesis of Compound **7-3**

N-cyclopentylformamide (2.79 g, 24.68 mmol) was dissolved in dichloromethane (55 mL), into which was added Burgess reagent (7.67 g, 32.19 mmol) at 20°C to react while stirring at 20°C for 1 hr. Water (1.93 mL) was added and stirred for 30 min. Compound **7-2** (5.5 g, 21.46 mmol) and acetic acid (2.45 mL) were added and the reaction solution was stirred at 20°C for 12 hr. 2.5% aqueous sodium hypochlorite solution (105.62 mL) was added into the reaction

solution and stirred for 30 min. The reaction solution was separated, and the aqueous phase was extracted with dichloromethane (50 mL×2). The organic phase was combined, washed with saturated brine (50 mL), dried over anhydrous sodium sulfate, and filtered. The filtrate was concentrated under reduced pressure, and the resulting crude product of Compound **7-3** was directly used in the next step.

Step **3:** Synthesis of Compound **7-4**

Compound **7-3** (2 g, 5.41 mmol) was dissolved in methanol (60 mL) and water (30 mL), into which was added potassium carbonate (5.04 g, 36.45 mmol) to react while stirring at 20°C for 2 hr. The reaction solution was adjusted to pH 5 to 6 with saturated citric acid solution, and then extracted with dichloromethane (100 mL). The organic phase was washed with saturated brine (20 mL), dried over anhydrous sodium sulfate, and filtered. The filtrate was concentrated under reduced pressure. The resulting crude product was added into ethyl acetate (50 mL), into which was dropwise added n-heptane (50 mL) while stirring for 2 hr. The solid was filtrated, and the filter cake was dried under vacuum to obtain Compound **7-4.**
$[M-100+1]^+$ = 269.9.

Step **4:** Synthesis of hydrochloride of Compound **7-5**

Compound **7-4** (10 g, 24.30 mmol) was dissolved in ethyl acetate (20 mL), into which was added a solution of hydrogen chloride in ethyl acetate (4 M, 5.41 mL) to react while stirring at 25°C for 12 hr. The reaction solution was filtered, and the filtrate was dried under vacuum to obtain a hydrochloride of Compound **7-5.** $[M+1]^+$ = 270.0.

Step **5:** Synthesis of Compound **7-7**

Compound **7-6** (2.00 g, 6.04 mmol) was dissolved in tetrahydrofuran (30 mL), into which were added zinc powder (3.25 g, 49.70 mmol), zirconocene dichloride (2.19 g, 7.24 mmol) and dibromomethane (1.15 g, 6.64 mmol). The reaction solution was heated to 80°C to react while stirring for 5 hr. Upon the reaction is completed, the reaction solution was cooled to room temperature, in which was added water (5 mL), and filtered. The filtrate was collected and extracted with methyl tert-butyl ether (100 mL×3). The organic phase was combined, dried over anhydrous sodium sulfate, and concentrated under reduced pressure. The resulting crude product was purified by flash silica gel chromatography (ISCO®; 12 g SepaFlash® flash silica gel column, eluant: 0 to 20% ethyl acetate/petroleum ether, flow rate: 30 mL/min), to obtain Compound **7-7.**
$[M+1]^+$ = 330.1.

Step **6:** Synthesis of Compound **7-8**

Under the protection of nitrogen, diethylzinc (1 M, THF, 37.95 mL) was added into dichloromethane (45 mL) and the reaction solution was stirred at 0°C. Trifluoroacetic acid (4.33 g, 37.95 mmol) was then added, and the reaction solution was stirred for 30 min. A solution of Compound **7-7** (2.50 g, 7.59 mmol) in dichloromethane (70 mL) was added, and the reaction solution was slowly warmed to 25°C and stirred for 16 hr. Saturated aqueous ammonium chloride solution (100 mL) was added, and the reaction solution was separated. The aqueous phase was extracted with dichloromethane (100 mL×3), and the organic phase was combined and concentrated under reduced pressure. The resulting crude product was purified by flash silica gel chromatography (ISCO®; 12 g SepaFlash® flash silica gel column, eluant: 0 to 20% ethyl acetate/petroleum ether, flow rate: 30 mL/min), to obtain Compound **7-8.** $[M+1]^+$ = 344.1.

Step 7: Synthesis of Compound **7-9**

Compound **7-8** (600.00 mg, 1.75 mmol) was dissolved in ethanol (30 mL), into which were added dioxane hydrochloride solution (4 M, 500.00 μL) and dry palladium on carbon (200 mg, 10% content) to react under hydrogen (15Psi) while stirring at 25°C for 12 hr. The reaction solution was filtered, the filtrate was collected and concentrated under reduced pressure to obtain Compound **7-9.** The crude product was directly used in the next step.

Step **8:** Synthesis of Compound **7-10**

Compound **7-9A** (852.41 mg, 3.69 mmol) and **7-9** (735.00 mg, 3.51 mmol) were dissolved in N,N-dimethylformamide (30 mL) and dichloromethane (40 mL), into which were added 2-(7-azabenzotriazole)-N,N,N',N'-tetramethyluronium hexafluophosphate (1.81 g, 4.77 mmol) and N,N-diisopropylethylamine (1.36 g, 10.53 mmol) to react under the protection of nitrogen while stirring at 25°C for 16 hr. The reaction solution was poured into 200 mL of 4M aqueous citric

acid solution and extracted with dichloromethane (100 mL×3). The organic phase was combined and concentrated under reduced pressure. The resulting crude product was purified by flash silica gel chromatography (ISCO®; 12 g SepaFlash® flash silica gel column, eluant: 0 to 20% ethyl acetate/petroleum ether, flow rate: 30 mL/min), to obtain Compound **7-10.** $[M+Na]^+$ = 445.2.

Step **9:** Synthesis of Compound **7-11**

Compound **7-10** (1.30 g, 3.08 mmol) was dissolved in tetrahydrofuran (6 mL), water (6 mL), and methanol (6 mL), into which was added lithium hydroxide monohydrate (387.30 mg, 9.23 mmol) to react while stirring at 25°C for 1 hr. The reaction solution was diluted in 100 mL of dichloromethane, into which was then added 50 mL of 1N hydrochloric acid aqueous solution and separated to extract the organic phase. The aqueous phase was extracted with dichloromethane (50 mL×3), and the organic phase was combined, dried over anhydrous sodium sulfate, and filtered. The filtrate was concentrated under reduced pressure to obtain Compound **7-11,** and the crude product was directly used in the next reaction. $[M-tBu+1]^+$ = 339.1.

Step **10:** Synthesis of hydrochloride of Compound **7-12**

Compound **7-11** (1.20 g, 3.04 mmol) was dissolved in tert-butyl methyl ether (10 mL), into which was added hydrogen chloride in ethyl acetate (4 M, 760.45 μL) to react while stirring at 25°C for 0.5 hr. The reaction solution was concentrated under reduced pressure to obtain a hydrochloride of Compound **7-12,** and the crude product was directly used in the next step. $[M+1]^+$ = 295.1.

Step **11:** Synthesis of Compound **7-13**

The hydrochloride of Compound **7-12** (1.20 g, 3.63 mmol) was dissolved in methanol (10 mL), into which were added triethylamine (1.84 g, 18.14 mmol) and ethyl trifluoroacetate (1.03 g, 7.25 mmol) to react while stirring at 25°C for 12 hr. The reaction solution was concentrated under reduced pressure, into which was added dichloromethane (100 mL), and then washed with 100 mL of 5% aqueous citric acid solution and separated for extraction. The aqueous phase was extracted with dichloromethane (200 mL×2), and the organic phase was combined, dried over anhydrous sodium sulfate, and concentrated to dry. The crude product was stirred in a mixed solvent of methyl tert-butyl ether and petroleum ether (1:6, 10 mL) at 25°C for 1 hr, and filtered to obtain a crude product of Compound **7-13,** which was directly used in the next step. $[M +1]^+$ = 390.9.

Step **12:** Synthesis of Compound **7-13A and 7-13B**

Compound **7-13** (100 mg, 256.15 μmol) was purified by preparative HPLC (chromatographic column: Xtimate C18 150*40mm*5μm; mobile phase: [A: water (hydrochloric acid)-B: acetonitrile]; acetonitrile%: 43%-63%, 10 min) to obtain **7-13A** (leaking peak, retention time: 4.198 min, $[M +1]^+$ = 391.2) and **7-13B** (tailing peak, retention time: 4.377 min, $[M +1]^+$ = 391.2). Analytical method: chromatographic column: ChromCore 120 C18 3 μm, 3.0*30 mm; mobile phase: [A: water (trifluoroacetic acid)-B: acetonitrile]; acetonitrile%: 10%-80% within 6 min, then holding at 80% for 0.5 min, flow rate: 0.8 mL/min.

Step **13:** Synthesis of Compound **7-14A**

Compound **7-13A** (35 mg, 89.65 μmol) and the hydrochloride of Compound **7-5** (41.12 mg) were dissolved in DCM (0.5 mL), into which were added 1-ethyl-(3-dimethylaminopropyl)carbodiimide hydrochloride (20.62 mg, 107.58 μmol), 2-hydroxypyridine-1-oxide (2.49 mg, 22.41 μmol) and N,N-diisopropylethylamine (34.76 mg, 268.96 μmol) to react while stirring at 25°C for 1 hr. The reaction solution was diluted with dichloromethane (5 mL), washed with saturated aqueous citric acid solution (1 mL) and saturated brine (1 mL) respectively, and dried over anhydrous sodium sulfate. The organic phase was concentrated to dry to obtain a crude product of Compound **7-14A,** which was directly used in the next step. $[M +1]^+$ = 642.2.

Step **14:** Synthesis of Compound **7-14B**

Compound **7-13B** (25.00 mg, 64.04 μmol) and the hydrochloride of Compound **7-5** (29.37 mg) were dissolved in DCM (0.5 mL), into which were added 1-ethyl-(3-dimethylaminopropyl)carbodiimide hydrochloride (14.73 mg, 76.84 μmol), 2-hydroxypyridine-1-oxide (1.78 mg, 16.01 μmol) and N,N-diisopropylethylamine (24.83 mg, 192.11 μmol) to react while stirring at 25°C for 1 hr. The reaction solution was diluted with dichloromethane (5 mL), washed with saturated

aqueous citric acid solution (1 mL) and saturated brine (1 mL) respectively, and dried over anhydrous sodium sulfate. The organic phase was concentrated to dry to obtain a crude product of Compound **7-14B,** which was directly used in the next step. [M +1]+ = 642.2.

Step **15:** Synthesis of Compound **7A**

Compound **7-14A** (50 mg, 77.92 μmol) was dissolved in DCM (0.5 mL), into which was added Dess-Martin periodinane (39.66 mg, 93.50 μmol) to react while stirring at 25°C for 1 hr. The reaction solution was diluted with dichloromethane (10 mL), washed with saturated aqueous sodium thiosulfate solution (2 mL), saturated aqueous sodium bicarbonate solution (2 mL) and saturated brine (2 mL) respectively, and dried over anhydrous sodium sulfate. The organic phase was concentrated to dry, and the resulting crude product was purified over silica gel column (petroleum ether: ethyl acetate = 1:1 to 0:1) to obtain Compound **7A.** [M +1]+ = 640.2.

Step **16:** Synthesis of Compound **7B**

Compound **7-14B** (35 mg, 54.54 μmol) was dissolved in DCM (0.5 mL), into which was added Dess-Martin periodinane (27.76 mg, 65.45 μmol) to react while stirring at 25°C for 1 hr. The reaction solution was diluted with dichloromethane (10 mL), washed with saturated aqueous sodium thiosulfate solution (2 mL), saturated aqueous sodium bicarbonate solution (2 mL) and saturated brine (2 mL) respectively, and dried over anhydrous sodium sulfate. The organic phase was concentrated to dry, and the resulting crude product was purified over silica gel column (petroleum ether: ethyl acetate = 1:1 to 0:1) to obtain Compound **7B.** $^{1}$H NMR (400 MHz, $CDCl_3$) δ ppm 8.95 (br d, J=4.02 Hz, 1H), 7.31 (br d, J=9.29 Hz, 1H), 6.76 (br d, J=7.78 Hz, 1H), 5.56 (s, 1H), 5.00-5.10 (m, 1H), 4.71 (d, J=9.03 Hz, 1H), 4.44 (s, 1H), 4.10-4.19 (m, 1H), 4.05 (t, J=9.91 Hz, 1H), 3.66 (dd, J=6.40, 10.67 Hz, 1H), 3.28-3.37 (m, 2H), 3.02 (q, J=7.11 Hz, 1H), 2.37-2.53 (m, 3H), 1.95-2.25 (m, 5H), 1.81-1.92 (m, 2H), 1.64-1.76 (m, 1H), 1.63-1.78 (m, 4H), 1.39-1.51 (m, 3H), 1.20-1.35 (m, 3H), 1.06-1.08 (m, 1H), 1.08 (s, 9H), 0.56-0.59 (m, 3H), 0.45-0.48 (m, 1H). [M +1]+ = 640.2.

**Biological Test**

**Test Example 1: Evaluation of in vitro inhibitory activity of the test compounds against Mpro protease of novel coronavirus**

1. Experimental materials:

1.1 Reagents, consumables and sources:

Tris: Sigma;
EDTA: Sigma;
NaCl: Sigma;
384 well Plate: Perkin Elmer;
Dimethyl sulfoxide (DMSO): Sigma;
Substrate (Dabcyl-KTSAVLQSGFRKM-(Edans)): GenScript;
SARS-CoV-2 Mpro: WuXi AppTec;
GC376: TargetMol.

1.2 Instruments and sources:

SpectraMax M2e Microplate Reader: Molecular Devices;
Echo 655 Liquid Handler: Labcyte;
Benchtop high speed centrifuge: Eppendorf.

2. Experimental method

The compounds were dissolved in DMSO and diluted in a 3-fold gradient using Echo655 according to the concentration required to be measured, with 10 concentration points and duplicate wells for each concentration, and

added to a 384-well plate. Mpro protein and a substrate were diluted with testing buffer (100 mM NaCl, 20 mM Tris-HCL, 1 mM EDTA), and the diluted Mpro protein was added to the 384-well test plate and incubated with the compounds at room temperature for 30 min. Then, the substrate was added, with the test concentration of the Mpro protein at 25 nM and the test concentration of the substrate at 25 μM, and the plate was incubated in a constant-temperature incubator at 30°C for 60 min. The microplate reader was then used to detect fluorescence signal values of Ex/Em=340nm/490nm. Simultaneously, a background well containing both the substrate and the compound but no Mpro protein was detected as control.

3. Data Analysis:

1) Inhibition rate was calculated using the formula below:

$$\text{Inhibition rate\%} = [(\text{Compound}-BG_{\text{Compound}})-(\text{ZPE}-BG_{\text{ZPE}})]/[(\text{HPE}-BG_{\text{HPE}})-(\text{ZPE}-BG_{\text{ZPE}})] * 100\%$$

# HPE: a control with 100% inhibition, containing 25 nM Mpro protein + 25 μM substrate + 1 μM GC376;
ZPE: a control without inhibition, containing 25 nM Mpro protein + 25 μM substrate, no compound;
Compound: a well with the test compound, containing 25 nM Mpro protein + 25 μM substrate + Compound;
BG: a background control well, containing 25μM substrate + Compound, no Mpro protein.

2) The inhibition rate data (inhibition rate%) of the compounds were analyzed by nonlinear fitting of log(agonist) vs. response -- Variable slope using a GraphPad Prism software, to obtain $IC_{50}$ values of the compounds, with the experimental results shown in Table 1.

**Table 1: In vitro inhibitory activity of the test compounds against novel coronavirus Mpro protease**

| **Compound No.** | $IC_{50}$ (nM) |
|---|---|
| 1 | 6 |
| 2 | 11 |
| 3 | 25 |
| 4 | 104 |
| 5 | 50 |
| 6 | 12 |
| **7A** | 15 |

Conclusion: the compounds of the present application exhibit excellent in vitro inhibitory activity against Mpro protease of novel coronavirus.

**Test Example 2: Evaluation of in vitro anti-coronavirus activity of the compounds using a cytopathic model**

1. Experimental materials

1.1. Reagents, consumables and sources

MEM Medium: Sigma;
L-Glutamine: Gibco;
Non-essential amino acid: Gibco;
Double antibody (Penicillin-Streptomycin Solution): HyClone;
Fetal Bovine Serum (FBS): ExCell;

Dulbecco's Phosphate-Buffered Saline (DPBS): Corning;
0.25% Trypsin: Gibco;
CellTiter Glo Cell Viability Assay Kit: Promega;
Remdesivir: MCE;
96-well plate: Grenier.

1.2. Instruments and sources

Microplate reader: BioTek;
Cell counter: Beckman;
$CO_2$ Incubator: Thermo.

1.3. Cells and Viruses

MRC5 cells and coronaviruses HCoV OC43 were both purchased from ATCC.

MRC5 cells were cultured in MEM (Sigma) culture medium supplemented with 10% fetal bovine serum (Excell), 1% double antibodies (Hyclone), 1% L-glutamine (Gibco) and 1% non-essential amino acids (Gibco). The MEM (Sigma) medium supplemented with 5% fetal bovine serum (Excell), 1% double antibodies (Hyclone), 1% L-glutamine (Gibco) and 1% non-essential amino acids (Gibco) was used as an experimental culture medium.

2. Experimental method

**Table 2: Virus test method used in this study**

| Viruses (strains) | Cells | Treatment time of Compounds (days) /End point method | Control compound | Assay reagents |
|---|---|---|---|---|
| HCoV OC43, 100TCID$_{50}$/well | 20,000 MRC5 cells/well | 5/CPE | Remdesivir | Cell Titer Glo. |

Cells were seeded into a 96-well microplate at a certain density (Table 2), and cultured overnight in a 5% $CO_2$, 37°C incubator. The next day, doubly diluted compounds, with 8 concentration points and duplicate wells, were added 50 μL per well. Then the diluted viruses were added to the cells at 100 TCID$_{50}$ per well, 50 μL per well. A cell control (cells, without compound treatment or viral infection), a virus control (cells infected with viruses, without compound treatment) and a medium control (only medium) were set. The final volume of the culture medium for this experiment was 200 μL, and the final concentration of DMSO in the culture medium was 0.5%, respectively. The cells were cultured for 5 days in a 5% $CO_2$, 33°C incubator. A cell viability assay kit CellTiter Glo (Promega) was used to detect the cell viability. The conditions for the cytotoxicity assay were the same as those for the antiviral assay, but without viral infection.

3. Data Analysis

The antiviral activity and cytotoxicity of the compounds were represented by the inhibition rate (%) of virus-induced cytopathic effects and cell viability (%) of the compounds at different concentrations, respectively. The calculation formulas were as below:

Inhibition rate (%) = (Readings of test wells - Mean value of virus control)/ (Mean value of

Cell viability (%) = (Readings of test wells - Mean value of medium control)/ (Mean value of cell control - Mean value of medium control) ×100.

Nonlinear fitting analysis was performed by GraphPad Prism on the inhibition rate and cell viability of the

compounds, and the half effective concentration (EC50) and half cytotoxic concentration (CC50) values of the compounds were calculated, with the experimental results shown in Table 3.

**Table 3: Evaluation of in vitro anti-coronavirus activity of the compounds using a cytopathic model**

| **Compound No.** | $EC_{50}$ (nM) | $CC_{50}$ (nM) |
|---|---|---|
| **1** | 259 | > 10000 |
| **2** | 368 | > 10000 |
| **3** | 146 | > 10000 |
| **4** | 823 | > 10000 |
| **5** | 368 | > 10000 |
| **7A** | 410 | > 10000 |

Conclusion: The compounds of the present application exhibit excellent in vitro anti-coronavirus activity at the cellular level and are not cytotoxic.

**Test Example 3: Pharmacokinetic test in mice**

In this study, C57BL/6J male mice were used as test animals, and the plasma concentrations at different time points in mice given the test compounds via intravenous injection and gavage were determined quantitatively by LC/MS/MS method to evaluate the pharmacokinetic profile of the test drugs in mice.

The solutions of the test compounds were given to mice (overnight fasting, 6-8-week-old) via oral administration or gavage. 40 μL of blood was collected from the saphenous vein of mice 0.083 hr, 0.25 hr, 0.5 hr, 1.0 hr, 2.0 hr, 4.0 hr, 8.0 hr, and 24.0 hr after intravenous administration to the animals, and placed in an anticoagulant tube added with EDTA-K2, and centrifuged at 4°C and at 3200 g for 10 min to collect plasma. After the plasma samples were treated, the plasma drug concentration was determined by the LC-MS/MS method. 40 μL of blood was collected from the saphenous vein of mice at 0.25 hr, 0.5 hr, 1.0 hr, 2.0 hr, 4.0 hr, 6.0 hr, 8.0 hr, and 24.0 hr after gavage administration to the animals, and placed in an anticoagulant tube added with EDTA-K2, and centrifuged at 4°C and at 3200 g for 10 min to collect the plasma. After the plasma samples were treated, the plasma drug concentration was determined by the LC-MS/MS method. The experimental results were shown in Table 4 and Table 5.

Table 4: Pharmacokinetic parameters in mice after intravenous injection

| **PF-07321332** (IV bolus 3 mg/kg) | | Compound **1** (IV bolus 3 mg/kg) | | Compound **3** (IV bolus 3 mg/kg) | |
|---|---|---|---|---|---|
| Time (h) | C (nmol/L) | Time (h) | C (nmol/L) | Time (h) | C (nmol/L) |
| 0.0830 | 11278 | 0.0830 | 6768 | 0.0830 | 6714 |
| 0.250 | 4321 | 0.250 | 5527 | 0.250 | 4735 |
| 0.500 | 967 | 0.500 | 3928 | 0.500 | 4266 |
| 1.00 | 107 | 1.00 | 1957 | 1.00 | 4452 |
| 2.00 | 14.4 | 2.00 | 867 | 2.00 | 2580 |
| 4.00 | 4.28 | 4.00 | 293 | 4.00 | 2014 |
| 8.00 | 5.84 | 8.00 | 89.7 | 8.00 | 1503 |
| 24.0 | ND | 24.0 | 6.59 | 24.0 | 223 |
| $T_{1/2}$ (h) | 0.71 | $T_{1/2}$ (h) | 3.80 | $T_{1/2}$ (h) | 6.24 |
| Cl (mL/min/kg) | 30.2 | Cl (mL/min/kg) | 10.1 | Cl (mL/min/kg) | 2.50 |
| Vdss (L/kg) | 0.456 | Vdss (L/kg) | 1.38 | Vdss (L/kg) | 1.27 |
| $AUC_{0\text{-}last}$ (h*nmol/L) | 3327 | $AUC_{0\text{-}last}$ (h*nmol/L) | 7789 | $AUC_{0\text{-}last}$ (h*nmol/L) | 30564 |

Table 5: Pharmacokinetic parameters in mice after gavage administration

| **PF-07321332** (PO 10 mg/kg) | | Compound **1** (PO 10 mg/kg) | | Compound **3** (PO 10 mg/kg) | |
|---|---|---|---|---|---|
| Time (h) | C (nmol/L) | Time (h) | C (nmol/L) | Time (h) | C (nmol/L) |
| 0.250 | 2410 | 0.250 | 533 | 0.250 | 1407 |
| 0.500 | 2340 | 0.500 | 706 | 0.500 | 2702 |
| 1.00 | 1189 | 1.00 | 952 | 1.00 | 4314 |
| 2.00 | 492 | 2.00 | 1287 | 2.00 | 4427 |
| 4.00 | 183 | 4.00 | 661 | 4.00 | 3006 |
| 6.00 | NA | 6.00 | 291 | 6.00 | 2042 |
| 8.00 | ND | 8.00 | 187 | 8.00 | 1506 |
| 24.0 | ND | 24.0 | 2.69 | 24.0 | 205 |
| $T_{1/2}$ (h) | 1.10 | $T_{1/2}$ (h) | 2.63 | $T_{1/2}$ (h) | 5.40 |
| $C_{max}$ (nmol/L) | 2715 | $C_{max}$ (nmol/L) | 1287 | $C_{max}$ (nmol/L) | 4806 |
| $T_{max}$ (h) | 0.292 | $T_{max}$ (h) | 2.00 | $T_{max}$ (h) | 1.50 |
| $AUC_{0\text{-}last}$ (h*nmol/L) | 3577 | $AUC_{0\text{-}last}$ (h*nmol/L) | 5698 | $AUC_{0\text{-}last}$ (h*nmol/L) | 33033 |
| F% | 32.7% | F% | 22% | F% | 32% |
| ND indicates not detected, NA indicates not applicable. Conclusion: the compounds of the present application have significantly higher exposure in plasma, slower clearance rates, longer half-lives and better pharmacokinetic properties than the reference molecule **PF-07321332.** | | | | | |

## Claims

**1.** A compound of formula (IV) or a pharmaceutically acceptable salt thereof,

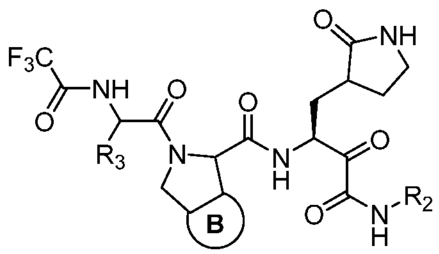

( IV)

wherein,

$R_3$ is selected from

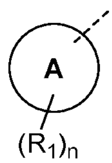

and $R_4$;

$R_1$ is each independently selected from F, Cl, Br, I, $OR_{11}$, CN, $CH_3S(O)_m$- and $NHR_{12}$, and $C_{1-3}$ alkyl, and the $C_{1-3}$ alkyl is optionally substituted with 1, 2 or 3 F;
$R_{11}$ is selected from H, $C_{1-3}$ alkyl, $CH_3(OCH_2CH2)_p$- and $H(OCH_2CH_2)_q$-, and the $C_{1-3}$ alkyl is optionally substituted with 1, 2 or 3 F;
$R_{12}$ is selected from $C_{1-3}$ alkyl, $CH_3CO$- and $CH_3SO_2$-, and the $C_{1-3}$ alkyl, $CH_3CO$- and $CH_3SO_2$- are optionally and independently substituted with 1, 2 or 3 F;
m is selected from 0, 1 and 2;
p and q are selected from 1, 2, 3, 4, 5, and 6;
n is selected from 0, 1, 2, 3, and 4;
$R_2$ is selected from $C_{1-4}$ alkyl, $C_{3-6}$ cycloalkyl and benzyl, and the $C_{1-4}$ alkyl, $C_{3-6}$ cycloalkyl and benzyl are optionally substituted with 1, 2 or 3 F;
$R_4$ is selected from $C_{1-8}$ alkyl optionally and independently substituted with 1, 2 or 3 F;
ring A is selected from $C_{3-10}$ cycloalkyl, 3-10-membered heterocycloalkyl and phenyl;
ring B is selected from $C_{3-8}$ cycloalkyl and 5-membered heterocycloalkyl, and the $C_{3-8}$ cycloalkyl and 5-membered heterocycloalkyl are optionally substituted with 1 or 2 $R_a$; and
$R_a$ is each independently selected from H and $C_{1-3}$ alkyl.

**2.** The compound or the pharmaceutically acceptable salt thereof according to claim 1, wherein, $R_1$ is selected from F and methyl.

**3.** The compound or the pharmaceutically acceptable salt thereof according to claim 1, wherein, ring A is selected from $C_{3-10}$ cycloalkyl.

**4.** The compound or the pharmaceutically acceptable salt thereof according to claim 3, wherein, ring A is selected from the group consisting of

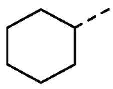 , 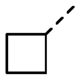 , 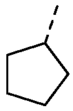 , 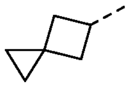 ,

and

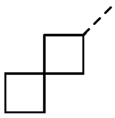 .

**5.** The compound or the pharmaceutically acceptable salt thereof according to claim 1, wherein, the structural unit

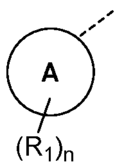

is selected from the group consisting of

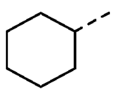 ,

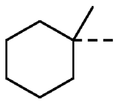, 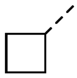, 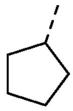, 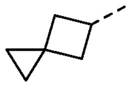, and 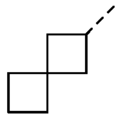.

6. The compound or the pharmaceutically acceptable salt thereof according to claim 1, wherein, $R_4$ is selected from t-butyl.

7. The compound or the pharmaceutically acceptable salt thereof according to claim 1, wherein, $R_a$ is selected from H and methyl.

8. The compound or the pharmaceutically acceptable salt thereof according to claim 1, wherein, ring B is selected from the group consisting of

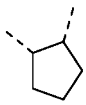 and 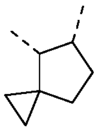.

9. The compound or the pharmaceutically acceptable salt thereof according to claim 1, wherein, the structural unit

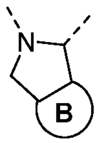

is selected from the group consisting of

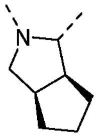, 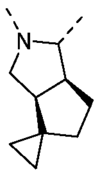,

and

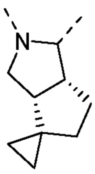.

10. The compound or the pharmaceutically acceptable salt thereof according to any one of claims 1-5, the compound is selected from structures of formulas (I-1), (IV-1) and (IV-2),

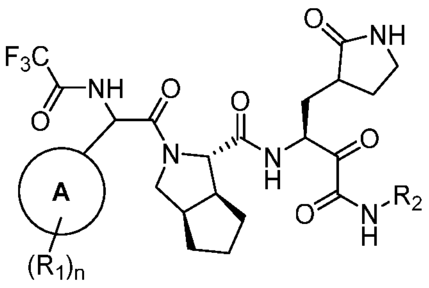

( I-1 ) ,

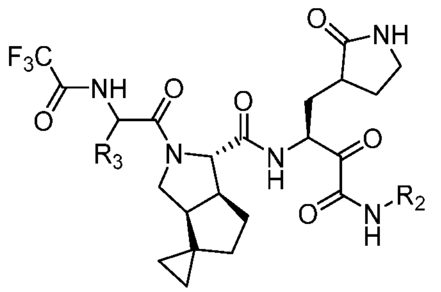

( IV-1 ) ,

and

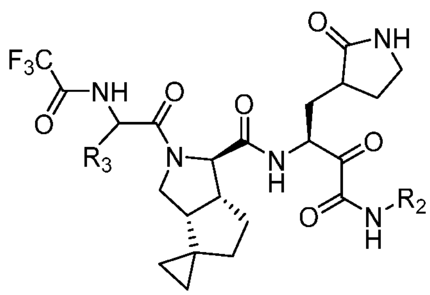

( IV-2 ) ,

wherein, $R_1$, $R_2$, $R_3$, n and ring A are as defined in any one of claims 1-5.

**11.** The compound or the pharmaceutically acceptable salt thereof according to claim 10, the compound is selected from structures of formulas (I-1a), (IV-1a) and (IV-2a),

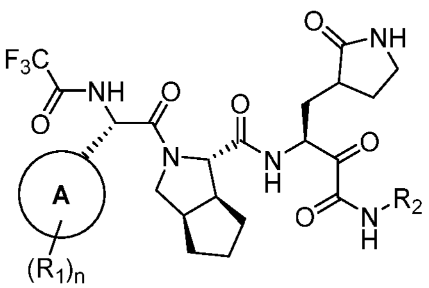

( I-1a ) ,

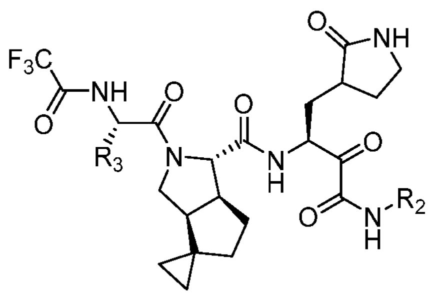

( IV-1a ) ,

and

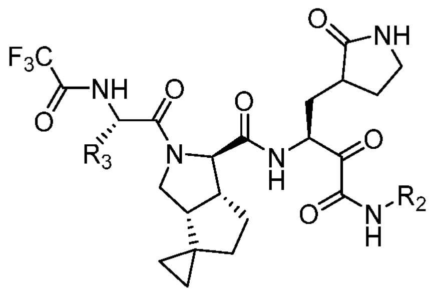

( IV-2a ) ,

wherein, $R_1$, $R_2$, $R_3$, n and ring A are as defined in claim 10.

**12.** A compound selected from the group consisting of the following formulas, or a pharmaceutically acceptable salt thereof,

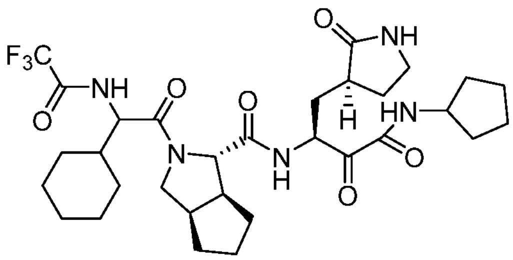

,

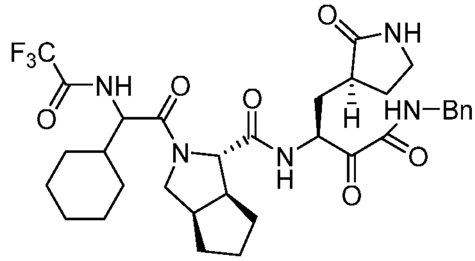

,

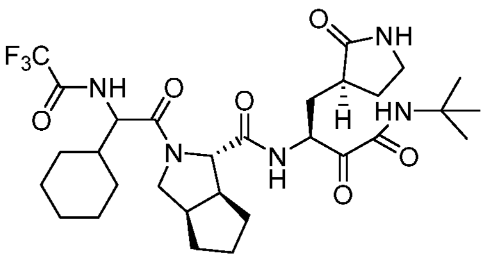

,

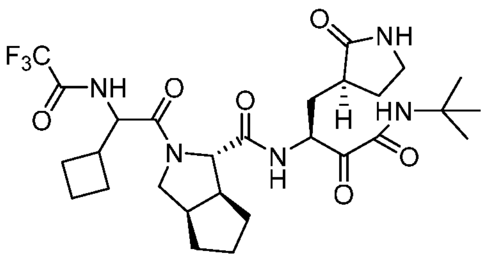

,

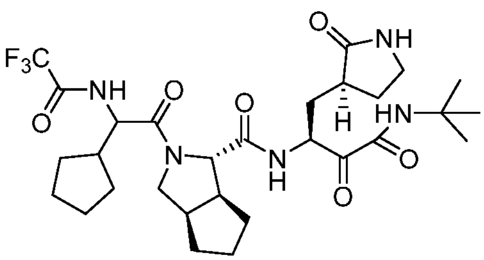

,

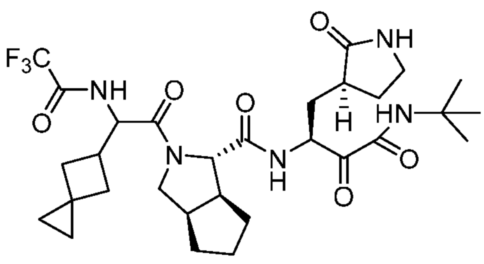

,

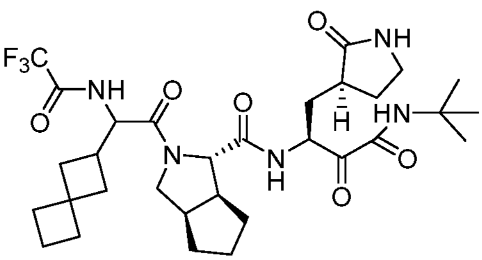

,

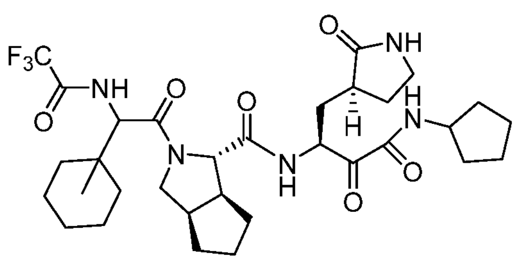

,

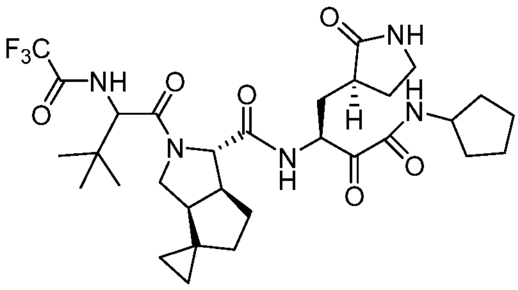

, and

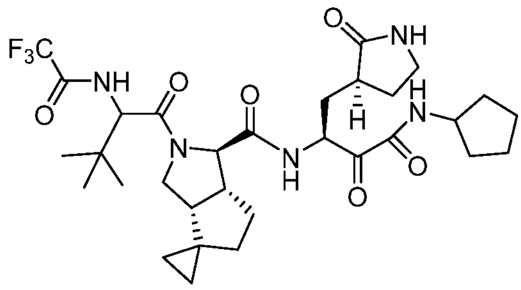

.

**13.** The compound or the pharmaceutically acceptable salt thereof according to claim 12, wherein the compound is selected from the group consisting of

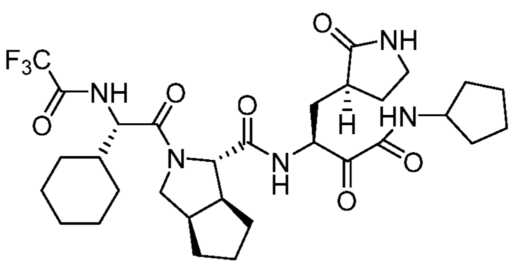

,

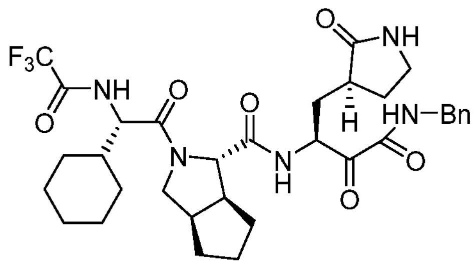

,

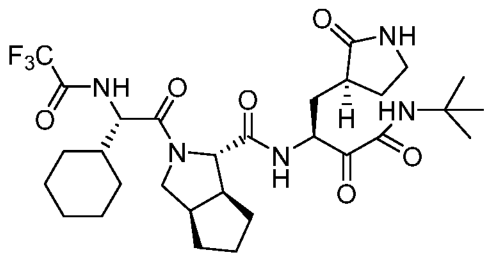

,

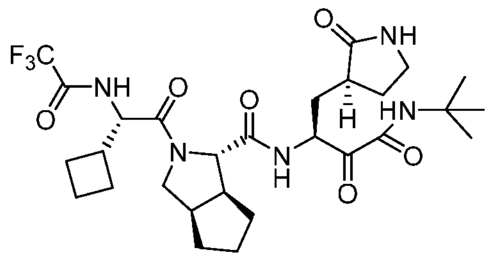

,

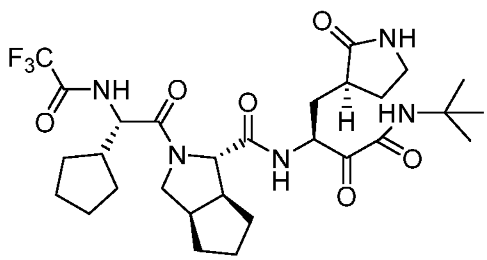

,

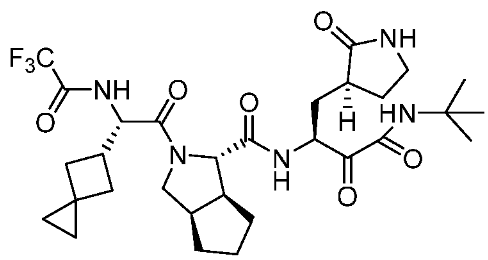

,

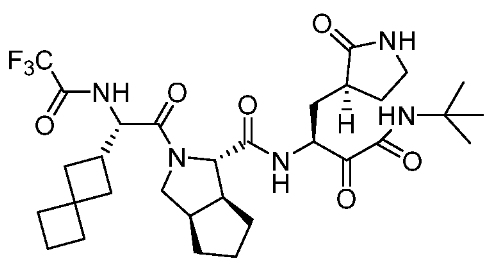

,

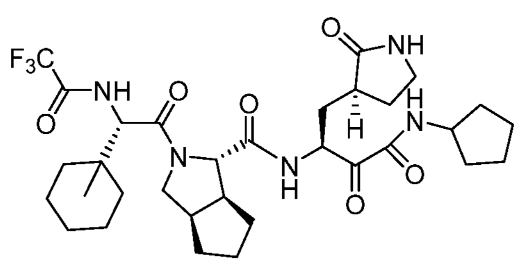

,

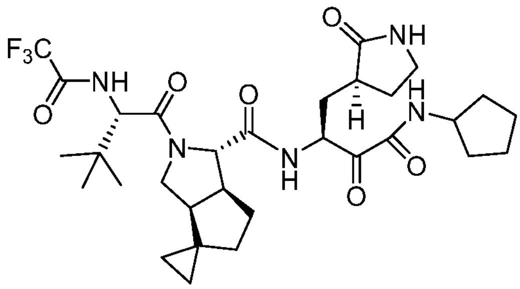

, and

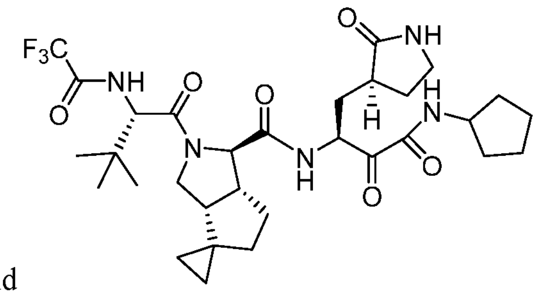

.

**14.** A method for treating a coronavirus infection, comprising administering to a subject in need thereof a therapeutically effective amount of the compound or the pharmaceutically acceptable salt thereof according to any one of claims 1-13, and a therapeutically acceptable dosage of further antiviral drug.

**15.** The method according to claim 14, wherein, the coronavirus is selected from an infection of HCoV-229E, HCoV-OC43, HCoV-NL63, HCoV-HKU1, SARS-CoV, MERS-CoV or SARS-CoV-2, or a variant thereof.

**16.** The method according to claim 14, wherein, the further antiviral drug is selected from the group consisting of Ritonavir, Indinavir, Nelfinavir, Saquinavir, Amprenavir or Lopinavir.

## INTERNATIONAL SEARCH REPORT

International application No.
**PCT/CN2022/117124**

**A. CLASSIFICATION OF SUBJECT MATTER**

C07D 413/12(2006.01)i； C07D 207/27(2006.01)i； A61K 31/4015(2006.01)i； A61P 31/00(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

CNABS, CNKI, EPODOC, WPI, REGISTRY(STN), CAPLUS(STN): 酮, 酰胺, 抑制剂, 新型冠状病毒, ketone, amide, inhibitor, SARS-CoV-2, COVID-19

**C. DOCUMENTS CONSIDERED TO BE RELEVANT**

| Category* | Citation of document, with indication, where appropriate, of the relevant passages | Relevant to claim No. |
|---|---|---|
| PX | WO 2021226546 A1 (THE BOARD OF TRUSTEES OF THE LELAND STANFORD JUNIOR UNIVERSITY) 11 November 2021 (2021-11-11) claims 15, 24, and 65-71, and description, p. 36, paragraph 5 | 1-16 |
| A | WESTBERG, M. et al. "Rational design of a new class of protease inhibitors for the potential treatment of coronavirus diseases." *BioRxiv*, 15 September 2020 (2020-09-15), pp. 1-19 | 1-16 |
| A | WO 2018042343 A2 (GLAXOSMITHKLINE INTELLECTUAL PROPERTY (NO.2) LIMITED) 08 March 2018 (2018-03-08) entire document | 1-16 |

☐ Further documents are listed in the continuation of Box C.  ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **04 November 2022** | **28 November 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2022/117124**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14-16**
because they relate to subject matter not required to be searched by this Authority, namely:

[1] The method of claims 14-16 is a method for the treatment of a disease; however, the present search report was made on the basis of a corresponding pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/117124**

| Patent document cited in search report | Publication date (day/month/year) | Patent family member(s) | Publication date (day/month/year) |
|---|---|---|---|
| WO 2021226546 A1 | 11 November 2021 | None | |
| WO 2018042343 A2 | 08 March 2018 | TW 201817714 A | 16 May 2018 |
| | | UY 37381 A | 23 March 2018 |

Form PCT/ISA/210 (patent family annex) (January 2015)